(19) Europäisches Patentamt
European Patent Office
Office européen des brevets

(11) Veröffentlichungsnummer: **0 132 680**
**B1**

(12) # EUROPÄISCHE PATENTSCHRIFT

(45) Veröffentlichungstag der Patentschrift: **28.01.87**

(51) Int. Cl.⁴: **C 07 D 271/08**, C 07 D 413/12, A 01 N 47/36

(21) Anmeldenummer: **84108004.7**

(22) Anmeldetag: **09.07.84**

(54) **Substituierte Furazane.**

(30) Priorität: **22.07.83 DE 3326509**
**27.02.84 DE 3407019**

(43) Veröffentlichungstag der Anmeldung:
**13.02.85 Patentblatt 85/7**

(45) Bekanntmachung des Hinweises auf die Patenterteilung:
**28.01.87 Patentblatt 87/5**

(84) Benannte Vertragsstaaten:
**AT BE CH DE FR GB IT LI NL**

(56) Entgegenhaltungen:
**CH - A - 508 650**

**CHEMICAL ABSTRACTS, Vol. 97, No. 11, 13. September 1982, Columbus, Ohio, USA. CALVINO, R.; FRUTTERO, R.; GASCO, A.; MORTARINI,V.;AIME, S.:"Unsymetrically substituted furoxans. VII. A carbon-13 NMR study of a series of isomeric pairs of furoxans and the structure of the two isomeric chlorophenylfuroxans" page 764, coiumn 2, abstract-No. 92 208c**
**CHEMICAL ABSTRACTS, vol. 94, No. 5, 2. Februar 1981, Columbus, Ohio, USA. CALVINO, ROSELLA; MORTARINI, VITTORIO; GASCO, ALBERTO; SANFILIPPO, AURORA; RICCIARDI, MARIA LUISA: "Antimicrobial properties of some furazan and furoxan**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 1, D-4322 Sprockhövel (DE)**
Erfinder: **Klauke, Erich, Dr., Eichendorffweg 8, D-5068 Odenthal (DE)**
Erfinder: **Zoebelein, Gerhard, Dr., Domblick 46, D-5090 Leverkusen 3 (DE)**
Erfinder: **Becker, Benedikt, Dr., Metzkausenerstrasse 14, D-4020 Mettmann (DE)**
Erfinder: **Stendel, Wilhelm, Dr., In den Birken 65, D-5600 Wuppertal 1 (DE)**

(56) Entgegenhaltungen: (Fortsetzung)
**derivatives", page 119, column 1, abstract No. 25 636r**
**J. Prakt.Chem.315(4) 791-795 (1973)**
**J.Chem.Soc.Perkin Trans I, 1977(14) 1616-1619**

**EP 0 132 680 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft substituierte Furazane, Verfahren zu ihrer Herstellung und ihre Verwendung als Schädlingsbekämpfungsmittel, insbesondere als Insektizide und Akarizide.

Es ist bisher noch nicht bekannt, dass substituierte Furazane als Schädlingsbekämpfungsmittel verwendet werden können, bisher ist lediglich bekannt, dass bestimmte Heterocyclen wie z.B. 3-Phenyl-2-phenylimino-4,5-bis-(trifluormethylimino)-thiazolidin, insektizide Eigenschaften besitzen (vgl. z.B. DE-AS 2 062 348). Bestimmte substituierte Furazane [N-(Phenyl)-N′-4-phenyl-1,2,5-oxadiazol-3-yl]-(thio)-harnstoff und [N-(4-Chlorphenyl)-N′-4-bromphenyl-1,2,5-oxadiazol-3-yl]-thioharnstoff sind bereits ohne die Angabe biologischer Eigenschaften beschrieben worden [vgl. J. Prakt. Chem. 315 (4), 791 - 795 (1973) und J. Chem. Soc. Perkin Trans. I, 1977 (14), 1616 - 1619].

Es wurde nun gefunden, dass die substituierten Furazane der Formel (I)

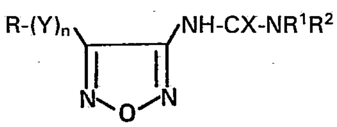

(I)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

$R^1$ für Reste aus der Reihe Phenyl, Pyridyl, Pyrimidinyl, 1,2,3-, 1,2,4- und 1,3,5-Triazinyl steht, welche substituiert sein können durch Reste aus der Reihe Halogen, Nitro, Cyano, $C_1$-$C_4$-Alkyl, $C_1$-$C_4$-Alkoxy, $C_1$-$C_6$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, $C_1$-$C_4$-Alkoxycarbonyl, Halogen-$C_1$-$C_4$-Alkoxycarbonyl, $C_1$-$C_4$-Alkoxycarbonyl-$C_1$-$C_2$-Alkyl, $C_1$-$C_4$-Alkoxycarbonyl-$C_1$-$C_4$-Alkylthio, $C_1$-$C_4$-Alkoxy-$C_1$-$C_4$-Alkylthio und/oder einen gegebenenfalls durch Fluor, Chlor und/oder Methyl substituierten $C_1$-$C_3$-Alkylen-Rest welcher 1 oder 2 Sauerstoffatome enthält, und/oder einen Phenoxy- oder Phenylthio-Rest, der seinerseits durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, $C_1$-$C_4$-Alkoxy-$C_1$-$C_2$-Alkyl, $C_1$-$C_4$-Alkylthio-$C_1$-$C_2$-Alkyl, $C_1$-$C_4$-Alkylsulfonyl, $C_1$-$C_4$-Alkoxycarbonyl und/oder einen gegebenenfalls durch Fluor, Chlor und/oder Methyl substituierten $C_1$-$C_3$-Alkylen-Rest, welcher 1 oder 2 Sauerstoffatome enthält, substituiert sein kann, und/oder einen Pyridin-2-yloxy-Rest, welcher seinerseits durch Halogen, $C_1$-$C_4$-Alkyl, Halogen-$C_1$-$C_2$-Alkoxy und/oder Halogen-$C_1$-$C_2$-Alkyl substituiert sein kann, und/oder einen Pyrimidin-5-yloxy- oder 1,3,5-Triazin-4-yloxy-Rest, welcher seinerseits durch $C_1$-$C_2$-Alkyl, $C_1$-$C_2$-Alkoxy, $C_1$-$C_2$-Alkylthio, Halogen-$C_1$-$C_2$-Alkyl, Halogen-$C_1$-$C_2$-Alkoxy, Halogen-$C_1$-$C_2$-Alkylthio, Fluor, Chlor und/oder Brom substituiert sein kann,

$R^2$ für Wasserstoff, eine OH- oder COOH-Gruppierung oder für gegebenenfalls durch Fluor, Chlor, $C_1$-$C_2$-Alkyl, $C_1$-$C_2$-Alkoxy, $C_1$-$C_2$-Alkylthio, Cyano und/oder Nitro substituierte Reste aus der Reihe $C_1$-$C_4$-Alkyl, $C_1$-$C_4$-Alkoxy, $C_3$-$C_6$-Cycloalkyl, $C_3$-$C_6$-Cycloalkenyl mit 1 oder 2 Doppelbindungen, $C_1$-$C_4$-Alkoxycarbonyl, $C_1$-$C_4$-Alkoxythiocarbonyl, $C_1$-$C_4$-Alkylsulfonyl, Phenylsulfonyl, Benzyl und Phenylethyl steht,

Y für Sauerstoff, eine $S(O)_m$-, $-CR^3R^4$-, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht,

als Schädlingsbekämpfungsmittel, insbesondere als Akarizide und Insektizide verwendet werden können.

Es wurden ferner die neuen substituierten Furazane der Formel (Ia) gefunden,

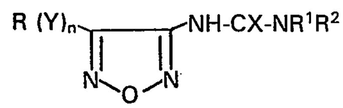

(Ia)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

$R^1$ für Reste aus der Reihe Phenyl, Pyridyl, Pyrimidinyl, 1,2,3-, 1,2,4- und 1,3,5-Triazinyl steht, welche substituiert sein können durch Reste aus der Reihe Halogen, Nitro, Cyano, $C_1$-$C_4$-Alkyl, $C_1$-$C_4$-Alkoxy, $C_1$-$C_6$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, $C_1$-$C_4$-Alkoxycarbonyl, Halogen-$C_1$-$C_4$-Alkoxycarbonyl, $C_1$-$C_4$-Alkoxycarbonyl-$C_1$-$C_2$-Alkyl, $C_1$-$C_4$-Alkoxycarbonyl-$C_1$-$C_4$-Alkylthio, $C_1$-$C_4$-Alkoxy-$C_1$-$C_4$-Alkylthio und/oder einen gegebenenfalls durch Fluor, Chlor und/oder Methyl substituierten $C_1$-$C_3$-Alkylen-Rest welcher 1 oder 2 Sauerstoffatome enthält, und/oder einen Phenoxy- oder Phenylthio-Rest, der seinerseits durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, $C_1$-$C_4$-Alkoxy-$C_1$-$C_2$-Alkyl, $C_1$-$C_4$-Alkylthio-$C_1$-$C_2$-Alkyl, $C_1$-$C_4$-Alkylsulfonyl, $C_1$-$C_4$-Alkoxycarbonyl und/oder einen gegebenenfalls durch Fluor, Chlor und/oder Methyl substituierten $C_1$-$C_3$-Alkylen-Rest, welcher 1 oder 2 Sauerstoffatome enthält, substituiert sein kann, und/oder einen Pyridin-2-yloxy-Rest, welcher seinerseits durch Halogen, $C_1$-$C_4$-Alkyl, Halogen-$C_1$-$C_2$-Alkoxy und/oder Halogen-$C_1$-$C_2$-Alkyl substituiert sein kann, und/oder einen Pyrimidin-5-yloxy- oder 1,3,5-Triazin-4-yloxy-Rest, welcher seinerseits durch $C_1$-$C_2$-Alkyl, $C_1$-$C_2$-Alkoxy, $C_1$-$C_2$-Alkylthio, Halogen-$C_1$-$C_2$-Alkyl, Halogen-$C_1$-$C_2$-Alkoxy, Halogen-$C_1$-$C_2$-Alkylthio, Fluor, Chlor und/oder Brom substituiert sein kann,

$R^2$ für Wasserstoff, eine OH- oder COOH-Gruppierung oder für gegebenenfalls durch Fluor, Chlor, $C_1$-$C_2$-Alkyl, $C_1$-$C_2$-Alkoxy, $C_1$-$C_2$-Alkylthio, Cyano und/oder Nitro substituierte Reste aus der Reihe $C_1$-$C_4$-Alkyl, $C_1$-$C_4$-Alkoxy, $C_3$-$C_6$-Cycloalkyl, $C_3$-$C_6$-Cycloalkenyl mit 1 oder 2 Doppelbindungen, $C_1$-$C_4$-Alkoxycarbonyl, $C_1$-$C_4$-Alkoxythiocarbonyl, $C_1$-$C_4$-Alkylsulfonyl, Phenylsulfonyl, Benzyl und Phenylethyl steht,

Y für Sauerstoff, eine $S(O)_m$-, $-CR^3R^4$-, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht,

wobei wenn

R für Phenyl steht,

$R^1$ für Phenyl steht,

$R^2$ für Wasserstoff steht, und

X für Sauerstoff oder Schwefel steht,

in diesen Fällen

n für die Zahl 1 steht oder

wenn

n für die Zahl 0 steht,

R für 4-Bromphenyl steht,

$R^1$ für 4-Chlorphenyl steht, und

$R^2$ für Wasserstoff steht,

in diesem Fall

X für Schwefel steht.

Weiterhin wurde gefunden, dass die neuen substituierten Furazane der Formel (Ia) erhalten werden, indem man

a) 3-Amino-1,2,5-oxadiazole der Formel (II)

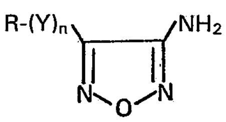

(II)

in welcher

Y, R und n die oben bei Formel Ia angegebenen Bedeutungen haben,

mit Iso(thio)cyanaten der Formel (III)

$R^1NCX$ (III)

in welcher

$R^1$ und X die oben bei Formel Ia angegebenen Bedeutungen haben,

gegebenenfalls in Gegenwart von Katalysatoren und gegebenenfalls in Gegenwart von Verdünnungsmitteln umsetzt, oder

b) 3-Iso(thio)cyanato-1,2,5-oxadiazole der Formel (IV),

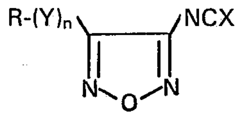

(IV)

in welcher

X, Y, R und n die oben bei Formel Ia angegebenen Bedeutungen haben,

mit Aminen der Formel (V),

$NHR^1R^2$ (V)

in welcher

$R^1$ und $R^2$ die oben bei Formel Ia angegebenen Bedeutungen haben,

gegebenenfalls in Gegenwart von Katalysatoren und gegebenenfalls in Gegenwart von Verdünnungsmitteln umsetzt.

Die neuen Verbindungen der Formel (Ia) verfügen über Eigenschaften, die ihre Verwendung als Schädlingsbekämpfungsmittel ermöglichen, insbesondere zeichnen sie sich durch eine hervorragende insektizide und akarizide Wirksamkeit aus. Zum Teil zeigen sie auch fungizide Wirkung insbesondere gegen Pyricularia oryzae und Pellicularia sasakii im Reis.

Bevorzugt werden die neuen substituierten Furazane der Formel (Ia), in welcher

R für Reste aus der Reihe Phenyl, Pyridin-2-yl, Pyrimidin-2-yl, 1,2,4-Triazin-2-yl und 1,3,5-Triazin-2-yl steht, welche substituiert sein können durch Fluor, Chlor, Brom, Cyano, Nitro, Methyl, Ethyl, Methoxy, Ethoxy, Methylthio, Trifluormethyl, Trifluormethoxy, Trifluormethylthio, Methylcarbonyl, Methoxycarbonyl, Ethoxycarbonyl, n-Butoxycarbonyl, tert.-Butoxycarbonyl, Methoxycarbonylmethyl, Ethoxycarbonylmethyl, Methoxycarbonylmethylthio und/oder Ethoxycarbonylmethylthio,

$R^1$ für Phenyl steht, welches substituiert sein kann durch Reste aus der Reihe Fluor, Chlor, Brom, Nitro, Cyano, Methoxy, Ethoxy, Methyl, Ethyl, n-Propyl, i-Propyl, n-Butyl, i-Butyl, sec.-Butyl, tert.-Butyl, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl, tert.-Butoxycarbonyl, Trifluorethoxycarbonyl, Methylcarbonyl, Methoxycarbonylmethyl, Methoxycarbonylethyl, Ethoxycarbonylmethyl, Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy, 2,2-Dimethylethylenoxy und/oder einen Phenoxy-Rest, der seinerseits gegebenenfalls durch Chlor, Brom, Nitro, Cyano, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-

Butoxy, tert.-Butoxy, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio, Methoxymethyl, Ethoxymethyl, Methylthiomethyl, Ethylthiomethyl Methoxycarbonyl, Ethoxycarbonyl, n-Propoxycarbonyl, i-Propoxycarbonyl, n-Butoxycarbonyl, i-Butoxycarbonyl, sec.-Butoxycarbonyl, tert.-Butoxycarbonyl, Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy und/oder 2,2-Dimethyl-ethylenoxy substituiert ist, und/oder einen Pyridin-2-yloxy-Rest, der seinerseits gegebenenfalls durch Chlor, Brom, Methyl, Trifluormethyl und/oder Trifluormethoxy substituiert ist, und/oder einen Pyrimidin-5-yl-oxy-Rest, welcher seinerseits gegebenenfalls durch Methyl, Methoxy, Methylthio, Trifluormethyl, Trifluormethoxy, Trifluormethylthio, Chlor und/oder Brom substituiert ist,

$R^2$ für Wasserstoff, Methyl, Ethyl, n-Propyl, i-Propyl, n-Butyl, i-Butyl oder sec.-Butyl steht,

Y für Sauerstoff, eine $S(O)_m$-, $CR^3R^4$-, CO- oder $NR^5$-Gruppierung steht, in welcher

m für 0, 1 oder 2 steht,

$R^3$, $R^4$ und $R^5$ für Wasserstoff oder Methyl stehen,

n für die Zahlen 0 oder 1 steht und

X für Sauerstoff oder Schwefel steht,

wobei wenn

R für Phenyl steht,

$R^1$ für Phenyl steht,

$R^2$ für Wasserstoff steht, und

X für Sauerstoff oder Schwefel steht,

in diesen Fällen

n für die Zahl 1 steht,

oder wenn

n für die Zahl 0 steht,

R für 4-Bromphenyl steht,

$R^1$ für 4-Chlorphenyl steht, und

$R^2$ für Wasserstoff steht,

in diesem Fall

X für Schwefel steht.

Von besonderem Interesse sind diejenigen Verbindungen der Formel (Ia), in welcher

R für gegebenenfalls durch Fluor, Chlor, Brom, Cyano, Nitro, Methyl, Methoxy und/oder Methylthio substituiertes Phenyl steht,

$R^1$ für einen Phenyl-Rest steht, welcher gegebenenfalls durch Chlor, Brom, Nitro, Cyano, Methoxy, Methylthio, Methyl, Trifluormethyl, Trifluormethoxy, Trifluormethylthio, Difluormethoxy, Chlordifluormethoxy, Chlortrifluorethoxy, Tetrafluorethoxy, Dichlordifluorethoxy, Difluortrichlorethoxy, Hexafluorn-propoxy, Difluormethylthio, Chlordifluormethylthio, Chlortrifluorethylthio, Hexafluorpropylthio, Ethoxycarbonyl, n-Propoxycarbonyl, n-Butoxycarbonyl, tert.-Butoxycarbonyl, Difluormethylendioxy, Chlortrifluorethylendioxy, Difluorethylendioxy, Trifluorethylendioxy, Tetrafluorethylendioxy, Difluormethylenoxydifluormethylenoxy, 2,2-Dimethyl-ethylenoxy und/oder einen Phenoxy-Rest, welcher seinerseits gegebenenfalls durch Chlor, Brom, Nitro, Cyano, Methyl, tert.-Butyl, Methoxy, Ethoxy, n-Propoxy, i-Propoxy, Trifluormethyl, Trifluormethoxy, Trifluormethylthio, Methoxymethyl, Methylthiomethyl, Ethoxymethyl, Ethylthiomethyl und/oder 2,2-Dimethylethylenoxy substituiert ist, und/oder einen Pyridin-2-yloxy-Rest, welcher seinerseits durch Chlor, Methyl, Methoxy, Trifluormethyl und/oder Trifluormethoxy substituiert sein kann,

$R^2$ für Wasserstoff oder Methyl steht,

Y für Sauerstoff, eine $S(O)_m$-, $CR^3R^4$-, CO- oder $NR^5$-Gruppierung steht, in welcher

m für 0, 1 oder 2 steht,

$R^3$, $R^4$ und $R^5$ für Wasserstoff oder Methyl stehen,

n für die Zahlen 0 oder 1 steht und

X für Sauerstoff oder Schwefel steht,

wobei wenn

R für Phenyl steht,

$R^1$ für Phenyl steht,

$R^2$ für Wasserstoff steht, und

X für Sauerstoff oder Schwefel steht,

in diesen Fällen

n für die Zahl 1 steht,

oder wenn

n für die Zahl 0 steht,

R für 4-Bromphenyl steht,

$R^1$ für 4-Chlorphenyl steht, und

$R^2$ für Wasserstoff steht,

in diesem Fall

X für Schwefel steht.

Von ganz besonderem Interesse sind diejenigen Verbindungen der Formel (Ia), in welcher

R für gegebenenfalls durch Fluor, Chlor, Brom, Cyano, Nitro, Methyl, Methoxy und/oder Methylthio substituiertes Phenyl steht,

$R^1$ für einen Phenyl-Rest steht, welcher gegebenenfalls substituiert ist durch Chlor, Trifluormethyl, Trifluormethylthio, Trifluormethoxy, Difluordichlorethoxy, Chlortrifluorethoxy, Tetrafluorethoxy, Difluortrichlorethoxy und/oder einen Phenoxy-Rest, welcher seinerseits gegebenenfalls durch Chlor, Nitro, Cyano, Methoxy, Ethoxy, n-Propoxy, i-Propoxy, Methyl, Trifluormethyl, Trifluormethoxy, Trifluormethylthio, Methoxymethyl, Methylthiomethyl, Ethylthiomethyl und/oder 2,2-Dimethyl-ethylenoxy substituiert ist, und/oder einen Pyridin-2-yloxy-Rest, welcher seinerseits gegebenenfalls durch Chlor, Trifluormethyl und/oder Trifluormethoxy substituiert ist,

$R^2$ für Wasserstoff steht,

n für die Zahl 0 steht und

X für Sauerstoff steht,

wobei wenn

R für Phenyl steht,

$R^1$ für Phenyl steht, und

$R^2$ für Wasserstoff steht,

in diesem Fall

n für die Zahl 1 steht,

oder wenn
R für 4-Bromphenyl steht,
$R^1$ für 4-Chlorphenyl steht, und
$R^2$ für Wasserstoff steht,

in diesem Fall
X für Schwefel steht.

Verwendet man nach Verfahrensvariante (a) 3-Amino-4-phenyl-1,2,5-oxadiazol und 4-(4-Chlorphenoxy)-phenylisocyanat als Ausgangsstoffe, so kann der Reaktionsablauf durch das folgende Formelschema wiedergegeben werden:

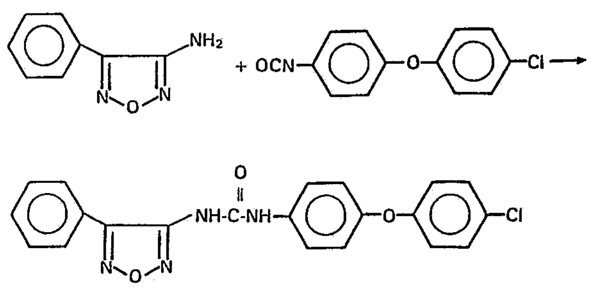

Verwendet man nach Verfahrensvariante (b) 4-(4-Chlorphenyl)-3-isocyanato-1,2,5-oxadiazol und 4-(4-Trifluormethylphenoxy)-anilin als Ausgangsstoffe, so kann der Reaktionsablauf durch das folgende Formelschema wiedergegeben werden:

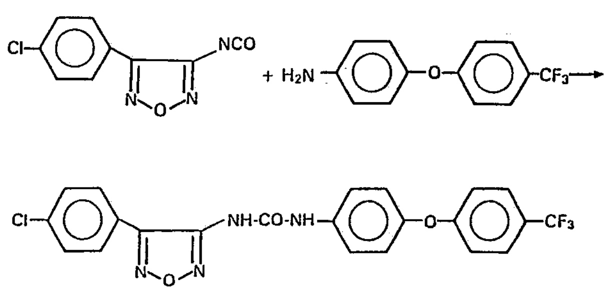

Die als Ausgangsstoffe zu verwendenden 3-Amino-1,2,5-oxadiazole der Formel (II) sind bekannt und/oder lassen sich nach literaturbekannten Verfahren und Methoden herstellen [vgl. J. Prakt. Chem. 315, 4, Seite 791 - 795 (1973)]. Die Aminogruppe kann nach üblichen Verfahren in die Isocyanat- bzw. Iso-thio-cyanat-Gruppe umgewandelt werden, z.B. durch Umsetzung mit Phosgen bzw. Thiophosgen in Verdünnungsmitteln wie z.B. Toluol und/oder Pyridin, bei einer Temperatur zwischen -20°C bis +50°C (vgl. Herstellungsbeispiel). Die Verbindungen der Formel (IV), die auf diese Weise erhalten werden, sind neu.

Zur vorliegenden Erfindung gehören die neuen Verbindungen der Formel (IV),

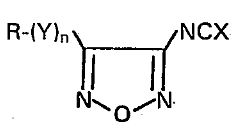

(IV)

in welcher
R, Y, n und X die oben bei Formel I angegebenen Bedeutungen haben.

Die Verbindungen der Formeln (III) und (V) sind bekannt und/oder lassen sich nach literaturbekannten Verfahren und Methoden herstellen (vgl. DE-OS 2 748 636, DE-OS 3 033 512 und EP 0 057 888).

Als Verdünnungsmittel kommen beim erfindungsgemässen Verfahren [Varianten (a) und (b)] praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzung gemäss Verfahrenvarianten (a) und (b) vorzugsweise tertiäre Amine, wie Triethylamin und 1,4-Diazabicyclo[2,2,2]-octan, sowie organische Zinn-Verbindungen, wie z.B. Dibutylzinndilaurat verwendet werden.

Die Reaktionstemperatur kann innerhalb eines grösseren Bereiches variiert werden. Im allgemeinen arbeitet man bei der Verfahrensvariante a) zwischen 20 und 180°C, vorzugsweise zwischen 40 und 120°C und bei der Verfahrensvariante b) zwischen 20 und 200°C, vorzugsweise zwischen 60 und 190°C. Die erfindungsgemässen Verfahrenvarianten werden im allgemeinen bei Normaldruck durchgeführt.

Zur Durchführung der erfindungsgemässen Verfahrensvarianten werden die Ausgangsstoffe gewöhnlich in etwa äquimolaren Mengen eingesetzt. Ein Überschuss der einen oder anderen Reaktionskomponente bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.

Aus der Ordnung der Thysanura z.B. Lepisma saccharina.
Aus der Ordnung der Collembola z.B. Onychiurus armatus.
Aus der Ordnung der Othoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsübliche Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemässen Wirkstoffe geschieht auf diesen Gebieten in bekannter Weise, wie durch äusserliche Anwendung in Form beispielsweise des Tauchens (Dippen), Sprühens (Sprayen), Aufgiessens (pour-on and spot-on) und des Einpuderns.

Die biologische Wirksamkeit der erfindungsgemässen Verbindungen soll anhand der folgenden Beispiele erläutert werden.

*Beispiel A*

Laphygma-Test

Lösungsmittel: 3 Gewichtsteile Dimethylformamid
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigte z.B. bei einer Konzentration von 0,1% beispielsweise die Verbindung des Herstellungsbeispiels (5) nach 7 Tagen eine Abtötung von 100%.

*Beispiel B*

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmässigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigte z.B. bei einer Konzentration von 3.000 ppm beispielsweise die Verbindung des Herstellungsbeispiels (8) eine Abtötung von 100%.

*Beispiel C*

*Eisterilitäts- und Entwicklungshemmtest mit Tetranychus urticae (Gemeine Spinnmilbe)*

Lösungsmittel: 3 Gewichtsteile Dimethylformamid
Emulgator: 1 Gewichtsteil Alkylarylpolyglykolether

Zur Herstellung einer zweckmässigen Wirkstoffzubereitung vermischt man 1 Gewichtsteil Wirkstoff

mit der angegebenen Menge Lösungsmittel und der angegebenen Menge Emulgator und verdünnt das Konzentrat mit Wasser auf die gewünschten Konzentrationen.

Die Blätter einer Bohnenpflanze (Phaseolus vulgaris) taucht man in die entsprechend konzentrierte Wirkstoffzubereitung. Nach Antrocknen der Wirkstoffzubereitung besetzt man die Blätter für ca. $16^h$ mit Spinnmilben-Weibchen zur Eiablage (ca. 50 Eier/Wiederholung). Die Summe der sterilen und/oder abgetöteten Eier und der abgetöteten Larven, Nymphen und Ruhestadien einer Generation bezogen auf die Zahl der abgelegten Eier ergibt die Abtötung in %. Dabei bedeutet 100%, dass alle Tiere abgetötet wurden; 0% bedeutet, dass keine Tiere abgetötet wurden.

Bei diesem Test zeigten z.B. bei einer Wirkstoffkonzentration von 0,02% die folgenden Verbindungen der Herstellungsbeispiele (1), (2), (5), (10), (20), (33), (34), (35), (38) und (82) nach 12 Tagen eine Abtötung von 100%.

*Beispiel D*

*Entwicklungsgemmtest mit Tetranychus urticae (Gemeine Spinnmilbe)*

Lösungsmittel: 3 Gewichtsteile Dimethylformamid
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Die Blätter der Bohnenpflanze (Phaseolus vulgaris), welche mit ca. 50 Eiern der Gemeinen Spinnmilbe belegt sind, tauch man in die entsprechend konzentrierte Wirkstoffzubereitung. Die Summe der abgetöteten Eier, Larven, Nymphen und Ruhestadien einer Generation bezogen auf die Zahl eingesetzter Eier ergibt die Abtötung in %. Dabei bedeutet 100%, dass alle Tiere abgetötet wurden; 0% bedeutet, dass keine Tiere abgetötet wurden.

Bei diesem Test zeigten z.B. bei einer Wirkstoffkonzentration von 0,02% die folgenden Verbindungen der Herstellungsbeispiele (1), (2), (5), (10), (20), (33), (34), (35), (38) und (82) nach 12 Tagen eine Abtötung von 100%.

Die Herstellung der erfindungsgemässen Verbindungen soll durch die folgenden Herstellungsbeispiele erläutert werden:

*Beispiel 1*

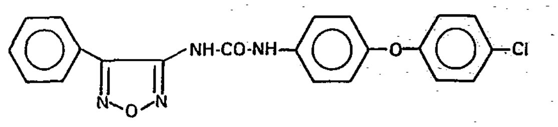

(Verfahrensvariante a)

3,23 g (0,02 Mol) 3-Amino-4-phenyl-1,2,5-oxadiazol werden bei 60°C in 100 ml trockenem Toluol angerührt. Anschliessend werden 4,92 g (0,02 Mol) 4-(4-Chlorphenoxy)-phenylisocyanat und ein Tropfen Dibutylzinndilaurat hinzugefügt, und das Gemisch wird 5 Stunden unter Rückfluss gekocht. Nach Abkühlung auf 20°C wird das auskristallisierte Produkt abgetrennt, mit Toluol nachgewaschen und getrocknet.

Man erhält 6,8 g (83,5% der Theorie N-[4-(4-Chlorphenoxy)-phenyl]-N'-4-phenyl-1,2,5-oxadiazol-3-yl)-harnstoff vom Schmelzpunkt 195°C.

*Beispiel 2*

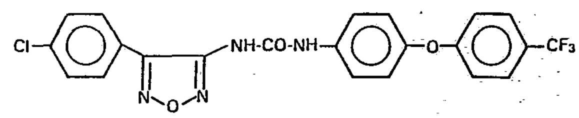

(Verfahrensvariante b)

2,53 g (0,01 Mol) 4-(4-Trifluormethylphenoxy)-anilin werden bei 60°C in 60 ml trockenem Toluol gelöst. Nach Zugabe von 2,2 g (0,01 Mol) 3-Isocyanato-4-(4-chlorphenyl)-1,2,5-oxadiazol in 10 ml trockenem Toluol wird 30 Minuten bei 80°C gerührt und anschliessend die Hauptmenge des Lösungsmittels abdestilliert. Nach Abtrennung des Rückstands wird getrocknet.

Man erhält 3,6 g (76%) der Theorie) N-[4-(4-Trifluormethylphenoxy)-phenyl]-N'-[4-(4-chlorphenyl)-1,2,5-oxadiazol-3-yl]-harnstoff vom Schmelzpunkt 208°C.

Analog Beispiel 1 od. 2 bzw. Verfahrensvariante (a) oder (b) werden die in der nachfolgenden Tabelle 1 aufgeführten Verbindungen der Formel (I) hergestellt:

Tabelle 1

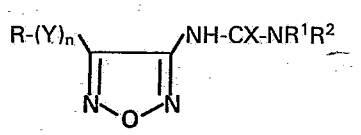

(I)

n = 0
X = O und
$R^2$ = H

| Beispiel Nr | R | $R^1$ | Schmelzpunkt °C |
|---|---|---|---|
| 3 | 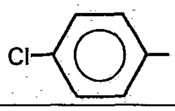 | 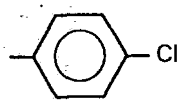 | 226 |

Tabelle 1 (Fortsetzung)

| Beispiel Nr | R | $R^1$ | Schmelzpunkt °C |
|---|---|---|---|
| 4 | 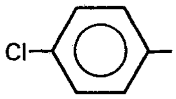 | 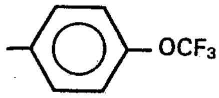 | 176 |
| 5 | 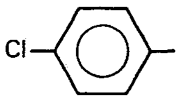 | 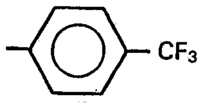 | 186 |
| 6 | 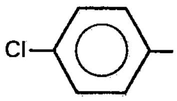 | 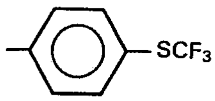 | 211 |
| 7 | 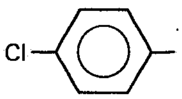 | 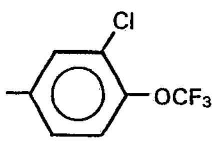 | 196 |
| 8 | 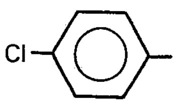 | 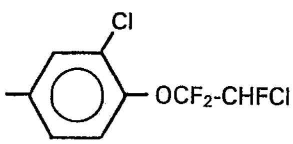 | 195 |
| 9 | 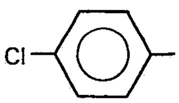 | 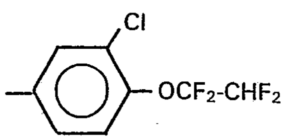 | 201 |
| 10 | 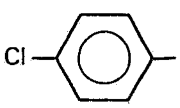 | 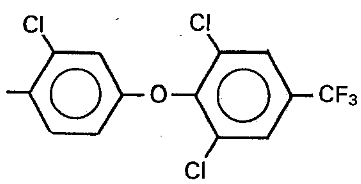 | 216 |
| 11 | 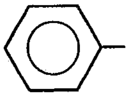 | 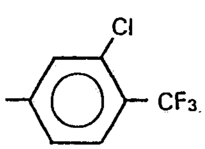 | 211 |
| 12 | 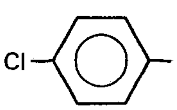 | 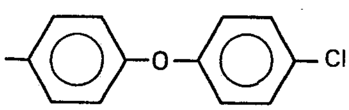 | 219 |
| 13 | 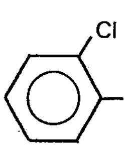 | 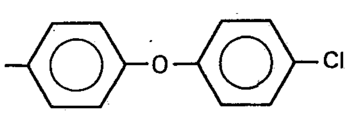 | 205 |

Tabelle 1 (Fortsetzung)

| Beispiel Nr | R | $R^1$ | Schmelzpunkt °C |
|---|---|---|---|
| 14 | 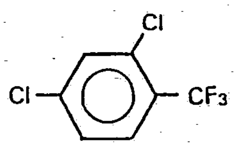 | 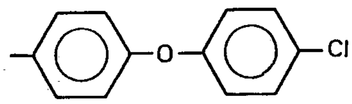 | 206 |
| 15 | 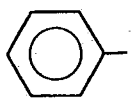 | 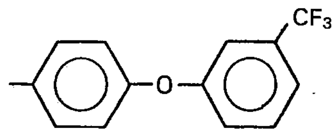 | 191 |
| 16 | 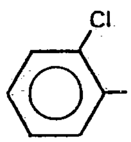 | 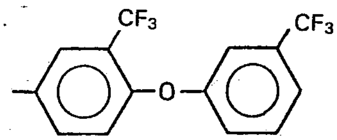 | 217 |
| 17 | 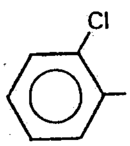 | 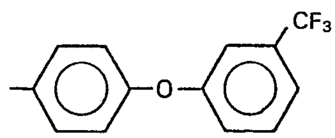 | 199 |
| 18 | 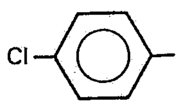 | 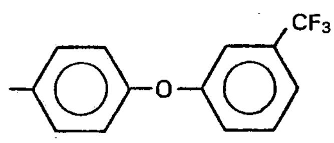 | 181 |
| 19 | 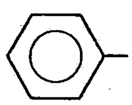 | 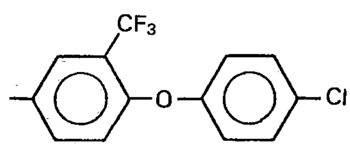 | 202 |
| 20 | 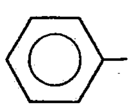 | 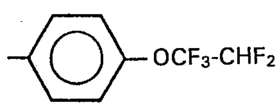 | 199 |
| 21 | 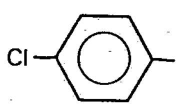 | 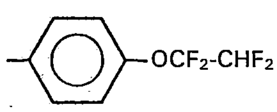 | 227 |
| 22 | 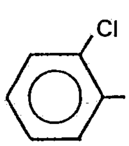 | 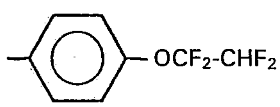 | 208 |

Tabelle 1 (Fortsetzung)

| Beispiel Nr | R | $R^1$ | Schmelzpunkt °C |
|---|---|---|---|
| 23 | 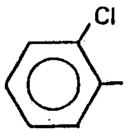 | 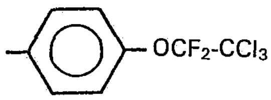 | 225 |
| 24 | 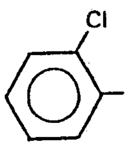 | 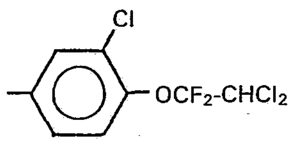 | 239 |
| 25 | 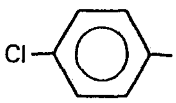 | 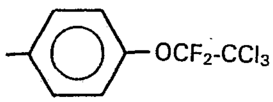 | 256 |
| 26 | 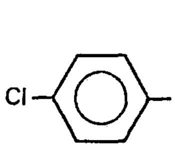 | 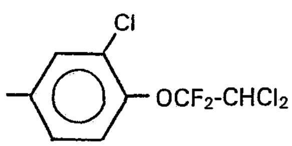 | 194 |
| 27 | 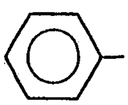 | 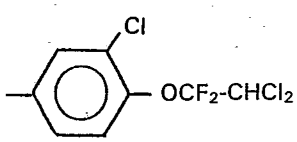 | 207 |
| 28 | 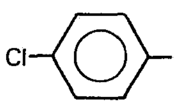 | 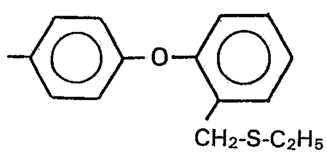 | 195 |
| 29 | 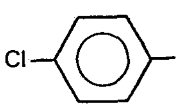 | 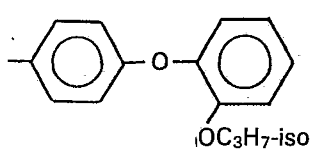 | 199 |
| 30 | 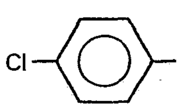 | 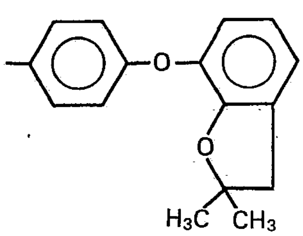 | 190 |
| 31 | 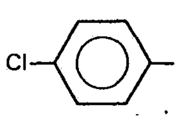 | 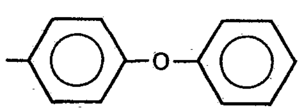 | 213 |

Tabelle 1 (Fortsetzung)

| Beispiel Nr | R | $R^1$ | Schmelzpunkt °C |
|---|---|---|---|
| 32 | 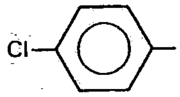 | 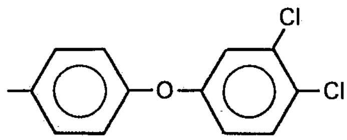 | 224 |
| 33 | 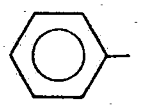 | 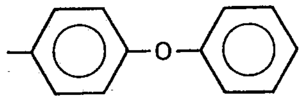 | 201 |
| 34 | 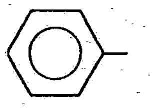 | 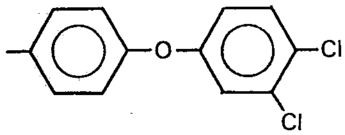 | 196 - 197 |
| 35 | 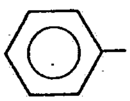 | 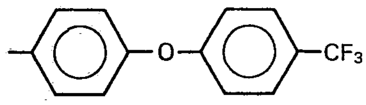 | 203 |
| 36 | 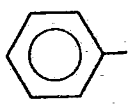 | 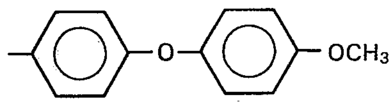 | 202 |
| 37 | 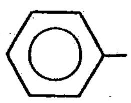 | 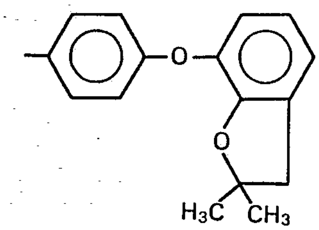 | 192 |
| 38 | 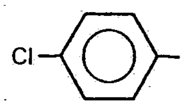 | 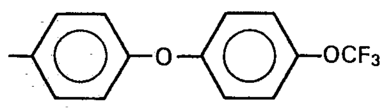 | 197 |
| 39 | 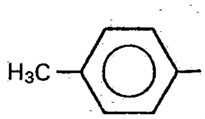 | 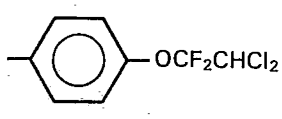 | 228 - 229 |
| 40 | 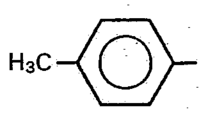 | 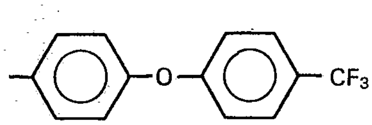 | 182 |
| 41 | 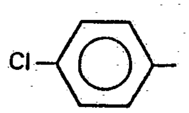 | 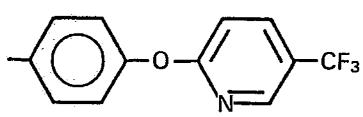 | 206 |

Tabelle 1 (Fortsetzung)

| Beispiel Nr | R | $R^1$ | Schmelzpunkt °C |
|---|---|---|---|
| 42 | 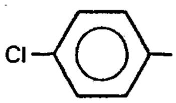 | 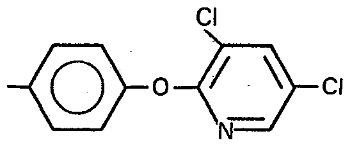 | 249 |
| 43 | 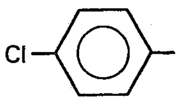 | 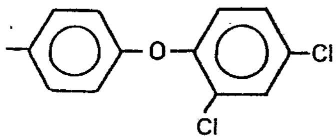 | 238 |
| 44 | 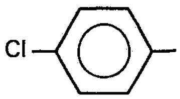 | 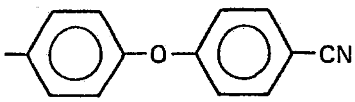 | 247 |
| 45 | 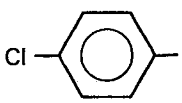 | 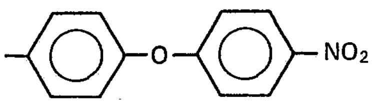 | 229 |
| 46 | 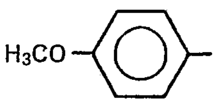 | 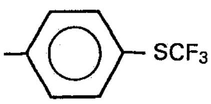 | 170 |
| 47 | 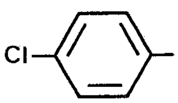 | 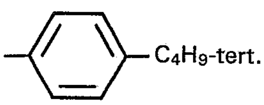 | 228 |
| 48 | 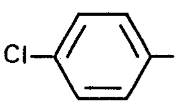 | 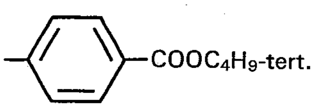 | 270 (Zers.) |
| 49 | 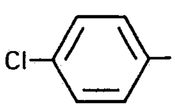 | 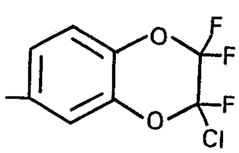 | 217 |
| 50 | 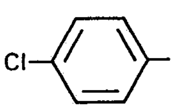 | 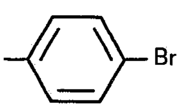 | 238 |
| 51 | 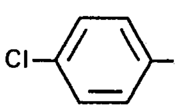 | 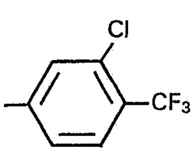 | 212 |
| 52 | 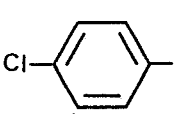 | 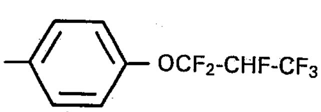 | 236 |

Tabelle 1 (Fortsetzung)

| Beispiel Nr | R | $R^1$ | Schmelzpunkt °C |
|---|---|---|---|
| 53 | 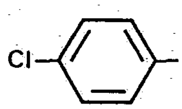 | 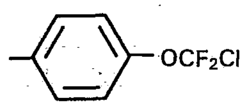 | 222 |
| 54 | 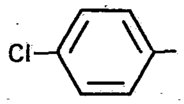 | 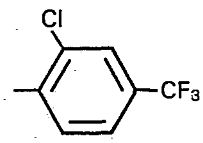 | 204 |
| 55 | 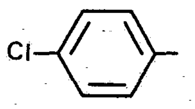 | 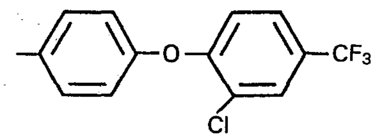 | 219 |
| 56 | 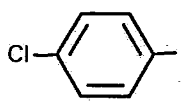 | 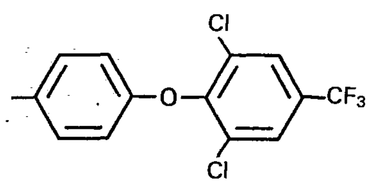 | 253 |
| 57 | 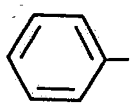 | 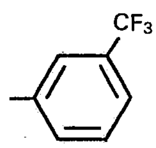 | 219 |
| 58 | 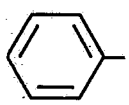 | 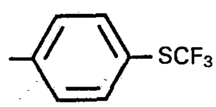 | 214 |
| 59 | 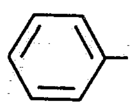 | 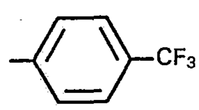 | 241 |
| 60 | 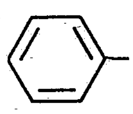 | 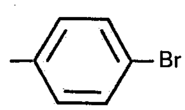 | 252 |
| 61 | 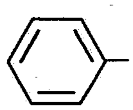 | 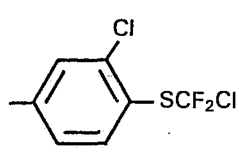 | 192 |
| 62 | 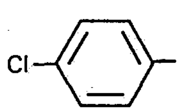 | 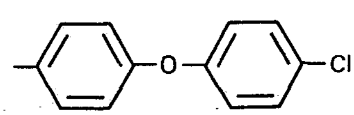 | 212 |

Tabelle 1 (Fortsetzung)

| Beispiel Nr | R | $R^1$ | Schmelzpunkt °C |
|---|---|---|---|
| 63 | 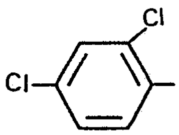 | 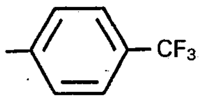 | 228 |
| 64 | 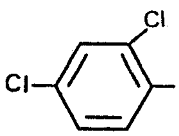 | 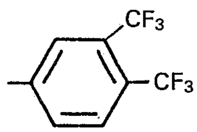 | 232 |
| 65 | 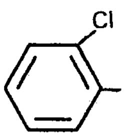 | 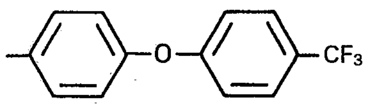 | 234 |
| 66 | 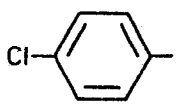 | 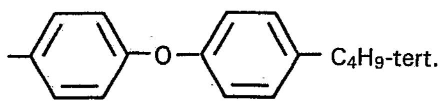 | 194 |
| 67 | 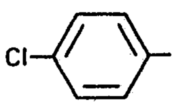 | 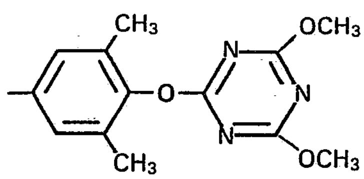 | 205 - 209 |
| 68 | 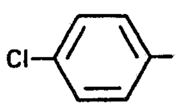 | 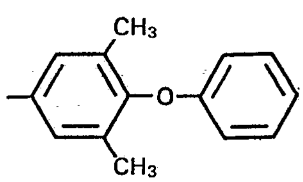 | 216 |
| 69 | 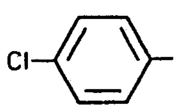 | 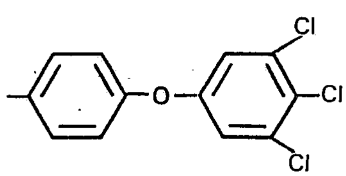 | 256 (Zers.) |
| 70 | 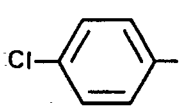 | 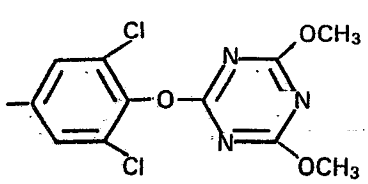 | 172 |

Tabelle 1 (Fortsetzung)

| Beispiel Nr | R | R[1] | Schmelzpunkt °C |
|---|---|---|---|
| 71 | 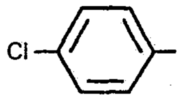 | 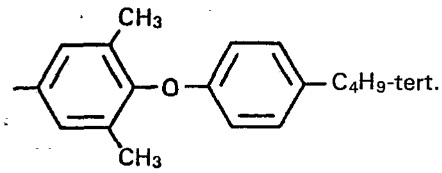 | 209 - 210 |
| 72 | 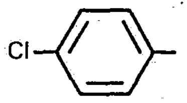 | 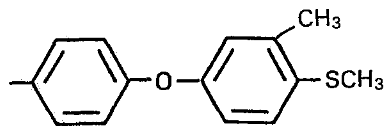 | 217 (Zers.) |
| 73 | 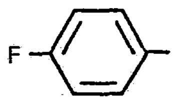 | 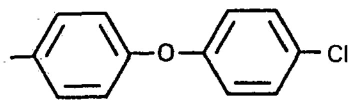 | 197 - 198 |
| 74 | 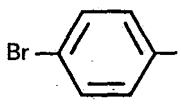 | 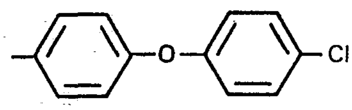 | 224 (Zers.) |
| 75 | 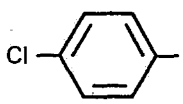 | 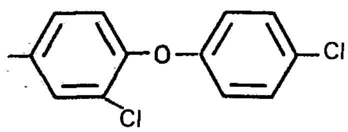 | 205 |
| 76 | 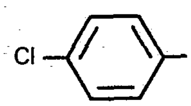 | 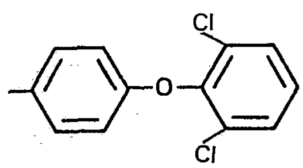 | 229 |
| 77 | 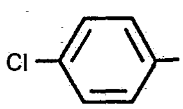 | 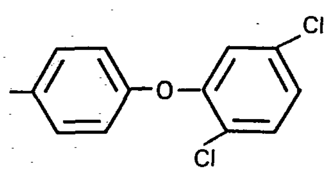 | 221 |
| 78 | 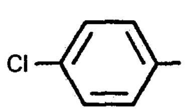 | 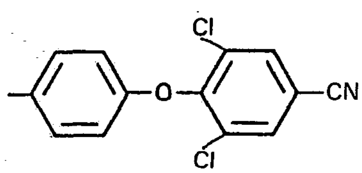 | 230 (Zers.) |

Tabelle 1 (Fortsetzung)

| Beispiel Nr | R | $R^1$ | Schmelzpunkt °C |
|---|---|---|---|
| 79 | 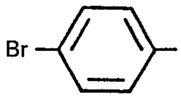 | 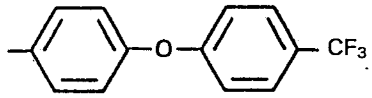 | 209 - 210 |
| 80 | 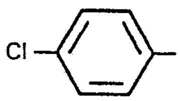 | 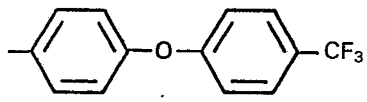 | 216 - 218 |
| 81 | 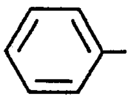 | 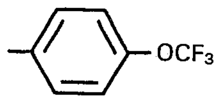 | 207 - 208 |
| 82 | 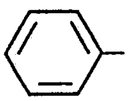 | 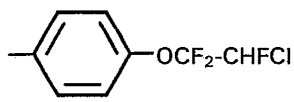 | 212 - 213 |
| 83 | 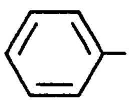 | 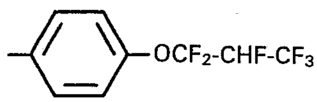 | 223 |
| 84 | 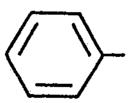 | 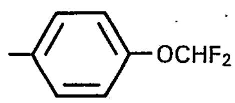 | 208 - 209 |
| 85 | 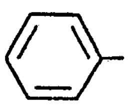 | 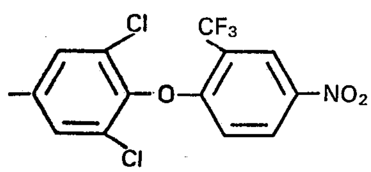 | 236 - 237 |
| 86 | 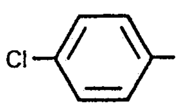 | 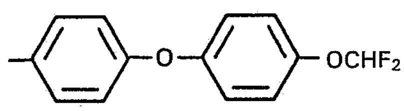 | 202 |
| 87 | 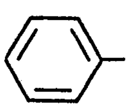 | 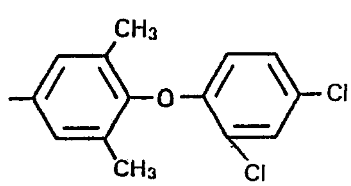 | 228 |
| 88 | 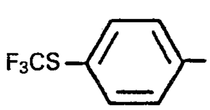 | 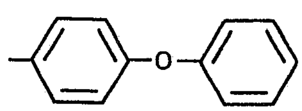 | 175 |

Tabelle 1 (Fortsetzung)

| Beispiel Nr | R | $R^1$ | Schmelzpunkt °C |
|---|---|---|---|
| 89 | 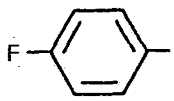 | 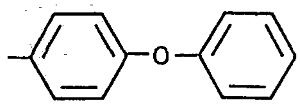 | 192 - 193 |
| 90 | 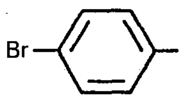 | 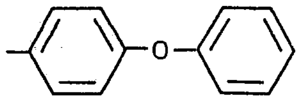 | 213 |
| 91 | 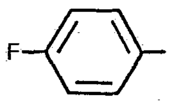 | 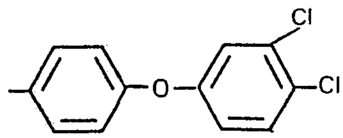 | 210 |
| 92 | 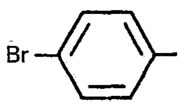 | 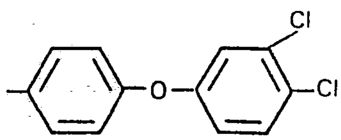 | 221 |
| 93 | 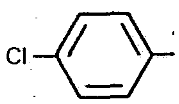 | 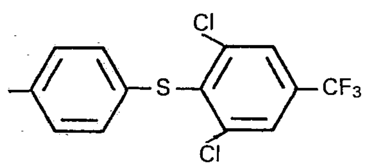 | 238 |
| 94 | 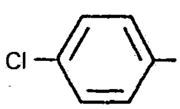 | 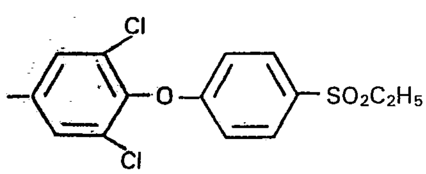 | 205 |
| 95 | 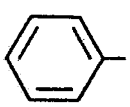 | 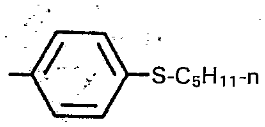 | 197 - 198 |
| 96 | 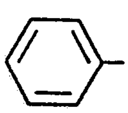 | 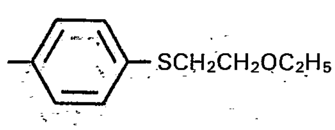 | 193 |

*Ausgangsprodukte der Formel (IV):*

*Beispiel (IV-1)*

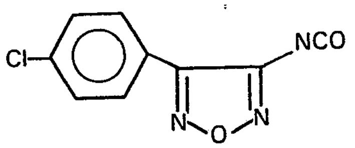

Bei 0°C bis 5°C werden 50 g Phosgen in 400 ml trockenem Toluol eingegast. In diese kalte Lösung werden bei −10°C bis +5°C eine Lösung von 58,7 g (0,3 Mol) 3-Amino-4-(4-chlorphenyl)-1,2,5-oxadiazol und 52,2 g (0,66 Mol) Pyridin in 400 ml Toluol zugetropft und 2 Stunden bei 30°C gerührt. Die ausgefallenen Salze werden unter Feuchtigkeitsausschluss abgesaugt, das Filtrat wird eingeengt und der Rückstand wird im Vakuum destilliert.

Man erhält 30,5 g (54,5% der Theorie) 4-(4-Chlorphenyl)-3-isocyanato-1,2,5-oxadiazol vom Siedepunkt $Kp_2$.: 108°C bis 112°C.

Analog Beispiel (IV-1) erhält man die übrigen Verbindungen der Formel (IV) wie beispielsweise:

3-Isocyanato-4-phenyl-1,2,5-oxadiazol (IV-2) vom Siedepunkt 85°C.

**Patentansprüche**

1. Substituierte Furazane der Formel (Ia)

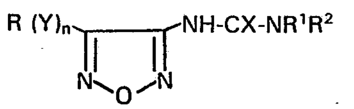

(Ia)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

$R^1$ für Reste aus der Reihe Phenyl, Pyridyl, Pyrimidinyl, 1,2,3-, 1,2,4- und 1,3,5-Triazinyl steht, welche substituiert sein können durch Reste aus der Reihe Halogen, Nitro, Cyano, $C_1$-$C_4$-Alkyl, $C_1$-$C_4$-Alkoxy, $C_1$-$C_6$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, $C_1$-$C_4$-Alkoxycarbonyl, Halogen-$C_1$-$C_4$-Alkoxycarbonyl, $C_1$-$C_4$-Alkoxycarbonyl-$C_1$-$C_2$-Alkyl, $C_1$-$C_4$-Alkoxycarbonyl-$C_1$-$C_4$-Alkylthio, $C_1$-$C_4$-Alkoxy-$C_1$-$C_4$-Alkylthio und/oder einen gegebenenfalls durch Fluor, Chlor und/oder Methyl substituierten $C_1$-$C_3$-Alkylen-Rest welcher 1 oder 2 Sauerstoffatome enthält, und/oder einen Phenoxy- oder Phenylthio-Rest, der seinerseits durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, $C_1$-$C_4$-Alkoxy-$C_1$-$C_2$-Alkyl, $C_1$-$C_4$-Alkylthio-$C_1$-$C_2$-Alkyl, $C_1$-$C_4$-Alkylsulfonyl, $C_1$-$C_4$-Alkoxycarbonyl und/oder einen gegebenenfalls durch Fluor, Chlor und/oder Methyl substituierten $C_1$-$C_3$-Alkylen-Rest, welcher 1 oder 2 Sauerstoffatome enthält, substituiert sein kann, und/oder einen Pyridin-2-yloxy-Rest, welcher seinerseits durch Halogen, $C_1$-$C_4$-Alkyl, Halogen-$C_1$-$C_2$-Alkoxy und/oder Halogen-$C_1$-$C_2$-Alkyl substituiert sein kann, und/oder einen Pyrimidin-5-yloxy- oder 1,3,5-Triazin-4-yloxy-Rest, welcher seinerseits durch $C_1$-$C_2$-Alkyl, $C_1$-$C_2$-Alkoxy, $C_1$-$C_2$-Alkylthio, Halogen-$C_1$-$C_2$-Alkyl, Halogen-$C_1$-$C_2$-Alkoxy, Halogen-$C_1$-$C_2$-Alkylthio, Fluor, Chlor und/oder Brom substituiert sein kann,

$R^2$ für Wasserstoff, eine OH- oder COOH-Gruppierung oder für gegebenenfalls durch Fluor, Chlor, $C_1$-$C_2$-Alkyl, $C_1$-$C_2$-Alkoxy, $C_1$-$C_2$-Alkylthio, Cyano und/oder Nitro substituierte Reste aus der Reihe $C_1$-$C_4$-Alkyl, $C_1$-$C_4$-Alkoxy, $C_3$-$C_6$-Cycloalkyl, $C_3$-$C_6$-Cycloalkenyl mit 1 oder 2 Doppelbindungen, $C_1$-$C_4$-Alkoxycarbonyl, $C_1$-$C_4$-Alkoxythiocarbonyl, $C_1$-$C_4$-Alkylsulfonyl, Phenylsulfonyl, Benzyl und Phenylethyl steht,

Y für Sauerstoff, eine $S(O)_m$-, $-CR^3R^4$-, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht,

wobei wenn

R für Phenyl steht,

$R^1$ für Phenyl steht,

$R^2$ für Wasserstoff steht, und

X für Sauerstoff oder Schwefel steht,

in diesen Fällen

n für die Zahl 1 steht oder

wenn

n für die Zahl 0 steht,

R für 4-Bromphenyl steht,

$R^1$ für 4-Chlorphenyl steht, und

$R^2$ für Wasserstoff steht,

in diesem Fall

X für Schwefel steht.

2. Substituierte Furazane der Formel (Ia) gemäss Anspruch 1, in welcher

R für Reste aus der Reihe Phenyl, Pyridin-2-yl, Pyrimidin-2-yl, 1,2,4-Triazin-2-yl und 1,3,5-Triazin-2-yl steht, welche substituiert sein können durch Fluor, Chlor, Brom, Cyano, Nitro, Methyl, Ethyl, Methoxy, Ethoxy, Methylthio, Trifluormethyl, Trifluormethoxy, Trifluormethylthio, Methylcarbonyl, Methoxycarbonyl, Ethoxycarbonyl, n-Butoxycarbonyl, tert.-Butoxycarbonyl, Methoxycarbonylmethyl, Ethoxycarbonylmethyl, Methoxycarbonylmethylthio und/oder Ethoxycarbonylmethylthio,

$R^1$ für Phenyl steht, welches substituiert sein kann durch Reste aus der Reihe Fluor, Chlor, Brom, Nitro, Cyano, Methoxy, Ethoxy, Methyl, Ethyl, n-Propyl, i-Propyl, n-Butyl, i-Butyl, sec.-Butyl, tert.-Butyl, Methylthio, Ethylthio, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio, Methoxycarbonyl, Ethoxycarbonyl, n-Propoxycarbonyl, i-Propoxycarbonyl, n-Butoxycarbonyl, i-Butoxycarbonyl, sec.-Butoxycarbonyl, tert.-Butoxycarbonyl, Trifluorethoxycarbonyl, Methylcarbonyl, Methoxycarbonylmethyl, Methoxycarbonylethyl, Ethoxycarbonylmethyl, Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy, 2,2-Dimethylethylenoxy und/oder einen Phenoxy-Rest, der seinerseits gegebenenfalls durch Chlor, Brom, Nitro, Cyano, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Trifluormethyl, Trifluormethoxy, Difluormethyl, Difluorethyl, Pentafluorethyl, Difluormethoxy, Chlordifluormethoxy, Chlortrifluorethoxy, Tetrafluorethoxy, Dichlordifluorethoxy, Difluortrichlorethoxy, Hexafluorpropoxy, Trifluormethylthio, Difluormethylthio, Chlordifluormethylthio, Chlortrifluorethylthio, Hexafluorpropylthio, Methoxymethyl, Ethoxymethyl, Methylthiomethyl, Ethylthiomethyl Methoxycarbonyl, Ethoxycarbonyl, n-Propoxycarbonyl, i-Propoxycarbonyl, n-Butoxycarbonyl, i-Butoxycarbonyl, sec.-Butoxycarbonyl, tert.-Butoxycarbonyl, Difluormethylendioxy, Ethylendioxy, Chlortrifluorethylendioxy, Difluorethylendioxy, Methylendioxy, Trifluorethylendioxy, Difluormethylenoxydifluormethylenoxy, Tetrafluorethylendioxy, 2-Methyl-1,1,2-trifluorethylendioxy und/oder 2,2-Dimethyl-ethylenoxy substituiert ist, und/oder einen Pyridin-2-yloxy-Rest, der seinerseits gegebenenfalls durch Chlor, Brom, Methyl, Trifluormethyl und/oder Trifluormethoxy substituiert ist, und/oder einen Pyrimidin-5-yl-oxy-Rest, welcher seinerseits gegebenenfalls durch Methyl, Methoxy, Methylthio, Trifluormethyl, Trifluormethoxy, Trifluormethylthio, Chlor und/oder Brom substituiert ist,

$R^2$ für Wasserstoff, Methyl, Ethyl, n-Propyl, i-Propyl, n-Butyl, i-Butyl oder sec.-Butyl steht,

Y für Sauerstoff, eine $S(O)_m$-, $CR^3R^4$-, CO- oder $NR^5$-Gruppierung steht, in welcher

m für 0, 1 oder 2 steht,

$R^3$, $R^4$ und $R^5$ für Wasserstoff oder Methyl stehen,

n für die Zahlen 0 oder 1 steht und

X für Sauerstoff oder Schwefel steht,

wobei wenn

R für Phenyl steht,

$R^1$ für Phenyl steht,

$R^2$ für Wasserstoff steht, und

X für Sauerstoff oder Schwefel steht,

in diesen Fällen

n für die Zahl 1 steht,

oder wenn

n für die Zahl 0 steht,

R für 4-Bromphenyl steht,

$R^1$ für 4-Chlorphenyl steht, und

$R^2$ für Wasserstoff steht,

in diesem Fall

X für Schwefel steht.

3. Verfahren zur Herstellung der neuen substituierten Furazane der Formel (Ia)

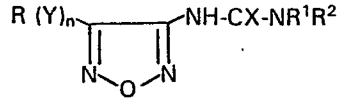

(Ia)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

$R^1$ für Reste aus der Reihe Phenyl, Pyridyl, Pyrimidinyl, 1,2,3-, 1,2,4- und 1,3,5-Triazinyl steht, welche substituiert sein können durch Reste aus der Reihe Halogen, Nitro, Cyano, $C_1$-$C_4$-Alkyl, $C_1$-$C_4$-Alkoxy, $C_1$-$C_6$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, $C_1$-$C_4$-Alkoxycarbonyl, Halogen-$C_1$-$C_4$-Alkoxycarbonyl, $C_1$-$C_4$-Alkoxycarbonyl-$C_1$-$C_2$-Alkyl, $C_1$-$C_4$-Alkoxycarbonyl-$C_1$-$C_4$-Alkylthio, $C_1$-$C_4$-Alkoxy-$C_1$-$C_4$-Alkylthio und/oder einen gegebenenfalls durch Fluor, Chlor und/oder Methyl substituierten $C_1$-$C_3$-Alkylen-Rest welcher 1 oder 2 Sauerstoffatome enthält, und/oder einen Phenoxy- oder Phenylthio-Rest, der seinerseits durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, $C_1$-$C_4$-Alkoxy-$C_1$-$C_2$-Alkyl, $C_1$-$C_4$-Alkylthio-$C_1$-$C_2$-Alkyl, $C_1$-$C_4$-Alkylsulfonyl, $C_1$-$C_4$-Alkoxycarbonyl und/oder einen gegebenenfalls durch Fluor, Chlor und/oder Methyl substituierten $C_1$-$C_3$-Alkylen-Rest, welcher 1 oder 2 Sauerstoffatome enthält, substituiert sein kann, und/oder einen Pyridin-2-yloxy-Rest, welcher seinerseits durch Halogen, $C_1$-$C_4$-Alkyl, Halogen-$C_1$-$C_2$-Alkoxy und/oder Halogen-$C_1$-$C_2$-Alkyl substituiert sein kann, und/oder einen Pyrimidin-5-yloxy- oder 1,3,5-Triazin-4-yloxy-Rest, welcher seinerseits durch $C_1$-$C_2$-Alkyl, $C_1$-$C_2$-Alkoxy, $C_1$-$C_2$-Alkylthio, Halogen-$C_1$-$C_2$-Alkyl, Halogen-$C_1$-$C_2$-Alkoxy, Halogen-$C_1$-$C_2$-Alkylthio, Fluor, Chlor und/oder Brom substituiert sein kann,

$R^2$ für Wasserstoff, eine OH- oder COOH-Gruppierung oder für gegebenenfalls durch Fluor, Chlor, $C_1$-$C_2$-Alkyl, $C_1$-$C_2$-Alkoxy, $C_1$-$C_2$-Alkylthio, Cyano und/oder Nitro substituierte Reste aus der Reihe $C_1$-$C_4$-Alkyl, $C_1$-$C_4$-Alkoxy, $C_3$-$C_6$-Cycloalkyl, $C_3$-$C_6$-Cycloalkenyl mit 1 oder 2 Doppelbindungen, $C_1$-$C_4$-Alkoxycarbonyl, $C_1$-$C_4$-Alkoxythiocarbonyl, $C_1$-$C_4$-Alkylsulfonyl, Phenylsulfonyl, Benzyl und Phenylethyl steht,

Y für Sauerstoff, eine $S(O)_m$-, $-CR^3R^4$-, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht,

wobei wenn

R für Phenyl steht,
$R^1$ für Phenyl steht,
$R^2$ für Wasserstoff steht, und
X für Sauerstoff oder Schwefel steht,

in diesen Fällen

n für die Zahl 1 steht oder

wenn

n für die Zahl 0 steht,
R für 4-Bromphenyl steht,
$R^1$ für 4-Chlorphenyl steht, und
$R^2$ für Wasserstoff steht,

in diesem Fall

X für Schwefel steht,

dadurch gekennzeichnet, dass man

a) 3-Amino-1,2,5-oxadiazole der Formel (II)

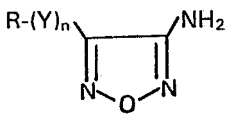

(II)

in welcher

Y, R und n die oben bei Formel Ia angegebenen Bedetungen haben,

mit Iso(thio)cyanaten der Formel (III)

$R^1NCX$ (III)

in welcher

$R^1$ und X die oben bei Formel Ia angegebenen Bedeutungen haben,

gegebenenfalls in Gegenwart von Katalysatoren und gegebenenfalls in Gegenwart von Verdünnungsmitteln umsetzt, oder

b) 3-Iso(thio)cyanato-1,2,5-oxadiazole der Formel (IV),

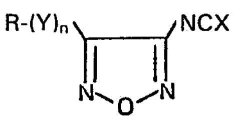

(IV)

in welcher

X, Y, R und n die oben bei Formel Ia angegebenen Bedeutungen haben,

mit Aminen der Formel (V),

$NHR^1R^2$ (V)

in welcher

$R^1$ und $R^2$ die oben bei Formel Ia angegebenen Bedeutungen haben,

gegebenenfalls in Gegenwart von Katalysatoren und gegebenenfalls in Gegenwart von Verdünnungsmitteln umsetzt.

4. Schädlingsbekämpfungsmittel, gekennzeichnet durch einen Gehalt an mindestens einem substituierten Furazan der Formel (Ia) gemäss Anspruch 1.

5. Verwendung von Verbindungen der Formel (I)

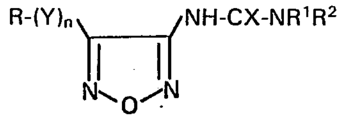

(I)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

$R^1$ für Reste aus der Reihe Phenyl, Pyridyl, Pyrimidinyl, 1,2,3-, 1,2,4- und 1,3,5-Triazinyl steht, welche substituiert sein können durch Reste aus der Reihe Halogen, Nitro, Cyano, $C_1$-$C_4$-Alkyl, $C_1$-$C_4$-Alkoxy, $C_1$-$C_6$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, $C_1$-$C_4$-Alkoxycarbonyl, Halogen-$C_1$-$C_4$-Alkoxycarbonyl, $C_1$-$C_4$-Alkoxycarbonyl-$C_1$-$C_2$-Alkyl, $C_1$-$C_4$-Alkoxycarbonyl-$C_1$-$C_4$-Alkylthio, $C_1$-$C_4$-Alkoxy-$C_1$-$C_4$-Alkylthio und/oder einen gegebenenfalls durch Fluor, Chlor und/oder Methyl substituierten $C_1$-$C_3$-Alkylen-Rest welcher 1 oder 2 Sauerstoffatome enthält, und/oder einen Phenoxy- oder Phenylthio-Rest, der seinerseits durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, $C_1$-$C_4$-Alkoxy-$C_1$-$C_2$-Alkyl, $C_1$-$C_4$-Alkylthio-$C_1$-$C_2$-Alkyl, $C_1$-$C_4$-Alkylsulfonyl, $C_1$-$C_4$-Alkoxycarbonyl und/oder einen gegebenenfalls durch Fluor, Chlor und/oder Methyl substituierten $C_1$-$C_3$-Alkylen-Rest, welcher 1 oder 2 Sauerstoffatome enthält, substituiert sein kann, und/oder einen Pyridin-2-yloxy-Rest, welcher seinerseits durch Halogen, $C_1$-$C_4$-Alkyl, Halogen-$C_1$-$C_2$-Alkoxy und/oder Halogen-$C_1$-$C_2$-Alkyl substituiert sein kann, und/oder einen Pyrimidin-5-yloxy- oder 1,3,5-Triazin-4-yloxy-Rest, welcher seinerseits durch $C_1$-$C_2$-Alkyl, $C_1$-$C_2$-Alkoxy, $C_1$-$C_2$-Alkylthio, Halogen-$C_1$-$C_2$-Alkyl, Halogen-$C_1$-$C_2$-Alkoxy, Halogen-$C_1$-$C_2$-Alkylthio, Fluor, Chlor und/oder Brom substituiert sein kann,

$R^2$ für Wasserstoff, eine OH- oder COOH-Gruppierung oder für gegebenenfalls durch Fluor, Chlor, $C_1$-$C_2$-Alkyl, $C_1$-$C_2$-Alkoxy, $C_1$-$C_2$-Alkylthio, Cyano und/oder Nitro substituierte Reste aus der Reihe $C_1$-$C_4$-Alkyl, $C_1$-$C_4$-Alkoxy, $C_3$-$C_6$-Cycloalkyl, $C_3$-$C_6$-Cycloalkenyl mit 1 oder 2 Doppelbindungen, $C_1$-$C_4$-Alkoxycarbonyl, $C_1$-$C_4$-Alkoxythiocarbonyl, $C_1$-$C_4$-Alkylsulfonyl, Phenylsulfonyl, Benzyl und Phenylethyl steht,

Y für Sauerstoff, eine $S(O)_m$-, $-CR^3R^4-$, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht, zur Bekämpfung von Schädlingen, insbesondere Insekten und Akariden.

6. Verfahren zur Bekämpfung von Schädlingen, dadurch gekennzeichent, dass man Verbindungen der Formel (I) gemäss Anspruch 5 auf die Schädlinge, vorzugsweise Insekten und Akariden, oder ihren Lebensraum einwirken lässt.

7. Verfahren zur Herstellung von Schädlingsbekämpfungsmitteln, dadurch gekennzeichnet, dass man Verbindungen der Formel (Ia) gemäss Anspruch 1 mit Streckmitteln und/oder oberflächenaktiven Mitteln vermischt.

8. Verbindungen der Formel (IV)

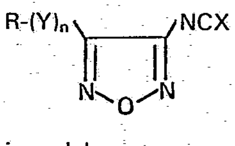

(IV)

in welcher

R für gegebenenfalls durch Halogen, Nitro, Cyano, $C_1$-$C_4$-Alkyl, $C_1$-$C_4$-Alkoxy, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Halogen-$C_1$-$C_4$-Alkylcarbonyl, $C_1$-$C_4$-Alkylcarbonyl, Halogen-$C_1$-$C_4$-Alkoxycarbonyl, $C_1$-$C_4$-Alkoxycarbonyl, $C_1$-$C_4$-Alkoxycarbonyl-$C_1$-$C_2$-Alkyl und/oder $C_1$-$C_4$-Alkoxycarbonyl-$C_1$-$C_4$-Alkylthio substituierte Reste aus der Reihe Phenyl, Pyridinyl, Pyrimidinyl, 1,2,3-, 1,2,4- und 1,3,5-Triazinyl steht,

Y für Sauerstoff, eine $S(O)_m$-, $-CR^3R^4$-, CO- oder $NR^5$-Gruppierung steht, in welcher m für die Zahlen 0, 1 oder 2 steht, $R^3$, $R^4$ und $R^5$ für Wasserstoff oder $C_1$-$C_4$-Alkyl stehen,

n für die Zahlen 0 oder 1 steht und

X für Sauerstoff oder Schwefel steht.

**Claims**

1. Substituted furazans of the formula (Ia)

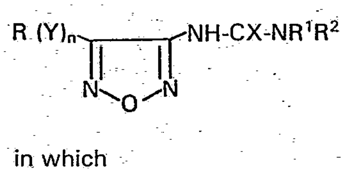

(Ia)

in which

R represents radicals which are optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkylcarbonyl, halogeno-$C_1$-$C_4$-alkoxycarbonyl, $C_1$-$C_4$-alkoxycarbonyl, $C_1$-$C_4$-alkoxycarbonyl-$C_1$-$C_2$-alkyl and/or $C_1$-$C_4$-alkoxycarbonyl-$C_1$-$C_4$-alkylthio and are chosen from the series comprising phenyl, pyridinyl, pyrimidinyl and 1,2,3-, 1,2,4- and 1,3,5-triazinyl,

$R^1$ represents radicals from the series comprising phenyl, pyridyl, pyrimidinyl and 1,2,3-, 1,2,4- and 1,3,5-triazinyl, which can be substituted by radicals from the series comprising halogen, nitro, cyano, $C_1$-$C_4$-alkyl, $C_1$-$C_4$-alkoxy, $C_1$-$C_6$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkyl-carbonyl, $C_1$-$C_4$-alkoxycarbonyl, halogeno-$C_1$-$C_4$-alkoxycarbonyl, $C_1$-$C_4$-alkoxycarbonyl-$C_1$-$C_2$-alkyl, $C_1$-$C_4$-alkoxycarbonyl-$C_1$-$C_4$-alkylthio, $C_1$-$C_4$-alkoxy-$C_1$-$C_4$-alkylthio, and/or a $C_1$-$C_3$-alkylene radical which contains 1 or 2 oxygen atoms and is optionally substituted by fluorine, chlorine and/or methyl, and/or a phenoxy or phenylthio radical, which can in turn be substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkyl-thio, $C_1$-$C_4$-alkoxy-$C_1$-$C_2$-alkyl, $C_1$-$C_4$-alkylthio-$C_1$-$C_2$-alkyl, $C_1$-$C_4$-alkylsulphonyl, $C_1$-$C_4$-alkoxycarbonyl and/or a $C_1$-$C_3$-alkylene radical which contains 1 or 2 oxygen atoms and is optionally substituted by fluorine, chlorine and/or methyl, and/or a pyridin-2-yloxy radical, which can in turn be substituted by halogen, $C_1$-$C_4$-alkyl, halogeno-$C_1$-$C_2$-alkoxy and/or halogeno-$C_1$-$C_2$-alkyl, and/or a pyrimidin-5-yloxy or 1,3,5-triazin-4-yloxy radical, which can in turn be substituted by $C_1$-$C_2$-alkyl, $C_1$-$C_2$-alkoxy, $C_1$-$C_2$-alkylthio, halogeno-$C_1$-$C_2$-alkyl, halogeno-$C_1$-$C_2$-alkoxy, halogeno-$C_1$-$C_2$-alkylthio, fluorine, chlorine and/or bromine,

$R^2$ represents hydrogen, an OH or COOH grouping, or radicals which are optionally substituted by fluorine, chlorine, $C_1$-$C_2$-alkyl, $C_1$-$C_2$-alkoxy, $C_1$-$C_2$-alkylthio, cyano and/or nitro and are chosen from the series comprising $C_1$-$C_4$-alkyl, $C_1$-$C_4$-alkoxy, $C_3$-$C_6$-cycloalkyl, $C_3$-$C_6$-cycloalkenyl with 1 or 2 double bonds, $C_1$-$C_4$-alkoxycarbonyl, $C_1$-$C_4$-alkoxythiocarbonyl, $C_1$-$C_4$-alkylsulphonyl, phenylsulphonyl, benzyl and phenylethyl,

Y represents oxygen or an $S(O)_m$, $-CR^3R^4$, CO or $NR^5$ grouping,

in which

m represents the numbers 0, 1 or 2 and

$R^3$, $R^4$ and $R^5$ represent hydrogen or $C_1$-$C_4$-alkyl,

n represents the numbers 0 or 1 and

X represents oxygen or sulphur,

wherein, if

R represents phenyl,

$R^1$ represents phenyl,

$R^2$ represents hydrogen and

X represents oxygen or sulphur,

then

n represents the number 1,

or if

n represents the number 0,

R represents 4-bromophenyl,

$R^1$ represents 4-chlorophenyl and

$R^2$ represents hydrogen,

then

X represents sulphur.

2. Substituted furazans of the formula (Ia) according to claim 1, in which

R represents radicals from the series comprising phenyl, pyridin-2-yl, pyrimidin-2-yl, 1,2,4-triazin-2-yl and 1,3,5-triazin-2-yl, which can be substituted by fluorine, chlorine, bromine, cyano, nitro, methyl, ethyl, methoxy, ethoxy, methylthio, trifluoromethyl, trifluoromethoxy, trifluoromethylthio, methylcarbonyl, methoxycarbonyl, ethoxycarbonyl, n-butoxycarbonyl, tert.-butoxycarbonyl, methoxycarbonylmethyl, ethoxycarbonylmethyl, methoxycarbonylmethylthio and/or ethoxycarbonylmethylthio,

$R^1$ represents phenyl, which can be substituted by radicals from the series comprising fluorine, chlorine, bromine, nitro, cyano, methoxy, ethoxy, methyl, ethyl, n-propyl, i-propyl, n-butyl, i-butyl, sec.-butyl, tert.-butyl, methylthio, ethylthio, trifluoromethyl, trifluoromethoxy, difluoromethyl, difluoroethyl, pentafluoroethyl, difluoromethoxy, chlorodifluoromethoxy, chlorotrifluoroethoxy, tetrafluoroethoxy, dichlorodifluoroethoxy, difluorotrichloroethoxy, hexafluoropropoxy, trifluoromethylthio, difluoromethylthio, chlorodifluoromethylthio, chlorotrifluoroethylthio, hexafluoropropylthio, methoxycarbonyl, ethoxycarbonyl, n-propoxycarbonyl, i-propoxycarbonyl, n-butoxycarbonyl, i-butoxycarbonyl, sec.-butoxycarbonyl, tert.-butoxycarbonyl, trifluoroethoxycarbonyl, methylcarbonyl, methoxycarbonylmethyl, methoxycarbonylethyl, ethoxycarbonylmethyl, difluoromethylenedioxy, ethylenedioxy, chlorotrifluoroethylenedioxy, difluoroethylenedioxy, methylenedioxy, trifluoroethylenedioxy, difluoromethyleneoxydifluoromethyleneoxy, tetrafluoroethylenedioxy, 2-methyl-1,1,2-trifluoroethylenedioxy, 2,2-dimethylethyleneoxy and/or a phenoxy radical, which is in turn optionally substituted by chlorine, bromine, nitro, cyano, methyl, ethyl, n-propyl, i-propyl, n-butyl, i-butyl, sec.-butyl, tert.-butyl, methoxy, ethoxy, n-propoxy, i-propoxy, n-butoxy, i-butoxy, sec.-butoxy, tert.-butoxy, trifluoromethyl, trifluoromethoxy, difluoromethyl, difluoroethyl, pentafluoroethyl, difluoromethoxy, chlorodifluoromethoxy, chlorotrifluoroethoxy, tetrafluoroethoxy, dichlorodifluoroethoxy, difluorotrichloroethoxy, hexafluoropropoxy, trifluoromethylthio, difluoromethylthio, chlorodifluoromethylthio, chlorotrifluoroethylthio, hexafluoropropylthio, methoxymethyl, ethoxymethyl, methylthiomethyl, ethylthiomethyl, methoxycarbonyl, ethoxycarbonyl, n-propoxycarbonyl, i-propoxycarbonyl, n-butoxycarbonyl, i-butoxycarbonyl, sec.-butoxycarbonyl, tert.-butoxycarbonyl, difluoromethylendioxy, ethylenedioxy, chlorotrifluoroethylenedioxy, difluoroethylenedioxy, methylenedioxy, trifluoroethylenedioxy, difluoromethyleneoxy-difluoromethyleneoxy, tetrafluoroethylenedioxy, 2-methyl-1,1,2-trifluoroethylenedioxy and/or 2,2-dimethyl-ethyleneoxy, and/or a pyridin-2-yloxy radical, which in turn is optionally substituted by chlorine, bromine, methyl, trifluoromethyl and/or trifluoromethoxy, and/or a pyrimidin-5-yl-oxy radical, which in turn is optionally substituted by methyl, methoxy, methylthio, trifluoromethyl, trifluoromethoxy, trifluoromethylthio, chlorine and/or bromine,

$R^2$ represents hydrogen, methyl, ethyl, n-propyl, i-propyl, n-butyl, i-butyl or sec.-butyl,

Y represents oxygen or an $S(O)_m$, $CR^3R^4$, CO or $NR^5$ grouping,

in which

m represents 0, 1 or 2 and

$R^3$, $R^4$ and $R^5$ represents hydrogen or methyl,

n represents the numbers 0 or 1 and

X represents oxygen or sulphur,

wherein, if

R represents phenyl,

$R^1$ represents phenyl,

$R^2$ represents hydrogen and

X represents oxygen or sulphur,

then

n represents the number 1,

or if

n represents the number 0,

R represents 4-bromophenyl,

$R^1$ represents 4-chlorophenyl and

$R^2$ represents hydrogen,

then

X represents sulphur.

3. Process for the preparation of the new substituted furazans of the formula (Ia)

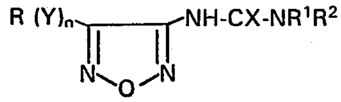

(Ia)

in which

R represents radicals which are optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkylcarbonyl, halogeno-$C_1$-$C_4$-alkoxycarbonyl, $C_1$-$C_4$-alkoxycarbonyl, $C_1$-$C_4$-alkoxycarbonyl-$C_1$-$C_2$-alkyl and/or $C_1$-$C_4$-alkoxycarbonyl-$C_1$-$C_4$-alkylthio and are chosen from the series comprising phenyl, pyridinyl, pyrimidinyl and 1,2,3-, 1,2,4- and 1,3,5-triazinyl,

$R^1$ represents radicals from the series comprising phenyl, pyridyl, pyrimidinyl and 1,2,3-, 1,2,4- and 1,3,5-triazinyl, which can be substituted by radicals from the series comprising halogen, nitro, cyano, $C_1$-$C_4$-alkyl, $C_1$-$C_4$-alkoxy, $C_1$-$C_6$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkyl-carbonyl, $C_1$-$C_4$-alkoxycarbonyl, halogeno-$C_1$-$C_4$-alkoxycarbonyl, $C_1$-$C_4$-alkoxycarbonyl-$C_1$-$C_2$-alkyl, $C_1$-$C_4$-alkoxycarbonyl-$C_1$-$C_4$-alkylthio, $C_1$-$C_4$-alkoxy-$C_1$-$C_4$-alkylthio, and/or a $C_1$-$C_3$-alkylene radical which contains 1 or 2 oxygen atoms and is optionally substituted by fluorine, chlorine and/or methyl, and/or a phenoxy or phenylthio radical, which can in turn be substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkyl-thio, $C_1$-$C_4$-alkoxy-$C_1$-$C_2$-alkyl, $C_1$-$C_4$-alkylthio-$C_1$-$C_2$-alkyl, $C_1$-$C_4$-alkylsulphonyl, $C_1$-$C_4$-alkoxycarbonyl and/or a $C_1$-$C_3$-alkylene radical which contains 1 or 2 oxygen atoms and is optionally substituted by fluorine, chlorine and/or methyl, and/or a pyridin-2-yloxy radical, which can in turn be substituted by halogen, $C_1$-$C_4$-alkyl, halogeno-$C_1$-$C_2$-alkoxy and/or halogeno-$C_1$-$C_2$-alkyl, and/or a pyrimidin-5-yloxy or 1,3,5-triazin-4-yloxy radical, which can in turn be substituted by $C_1$-$C_2$-alkyl, $C_1$-$C_2$-alkoxy, $C_1$-$C_2$-alkylthio, halogeno-$C_1$-$C_2$-alkyl, halogeno-$C_1$-$C_2$-alkoxy, halogeno-$C_1$-$C_2$-alkylthio, fluorine, chlorine and/or bromine,

$R^2$ represents hydrogen, an OH or COOH grouping, or radicals which are optionally substituted by fluorine, chlorine, $C_1$-$C_2$-alkyl, $C_1$-$C_2$-alkoxy, $C_1$-$C_2$-alkylthio, cyano and/or nitro and are chosen from the series comprising $C_1$-$C_4$-alkyl, $C_1$-$C_4$-alkoxy, $C_3$-$C_6$-cycloalkyl, $C_3$-$C_6$-cycloalkenyl with 1 or

2 double bonds, $C_1$-$C_4$-alkoxycarbonyl, $C_1$-$C_4$-alkoxythiocarbonyl, $C_1$-$C_4$-alkylsulphonyl, phenylsulphonyl, benzyl and phenylethyl,

Y represents oxygen or an $S(O)_m$, $-CR^3R^4$, CO or $NR^5$ grouping,

in which

m represents the numbers 0, 1 or 2 and

$R^3$, $R^4$ and $R^5$ represent hydrogen or $C_1$-$C_4$-alkyl,

n represents the numbers 0 or 1 and

X represents oxygen or sulphur,

wherein, if

R represents phenyl,

$R^1$ represents phenyl,

$R^2$ represents hydrogen and

X represents oxygen or sulphur,

then

n n represents the number 1

or if

n represents the number 0,

R represents 4-bromophenyl,

$R^1$ represents 4-chlorophenyl and

$R^2$ represents hydrogen,

then

X represents sulphur,

characterised in that

a) 3-amino-1,2,5-oxadiazoles of the formula (II)

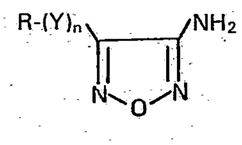

(II)

in which

Y, R and n have the meanings given above in the case of formula Ia,

are reacted with iso(thio)cyanates of the formula (III)

$R^1NCX$ (III)

in wich

$R^1$ and X have the meanings given above in the case of formula Ia,

if appropriate in the presence of catalysts and if appropriate in the presence of diluents, or

b) 3-iso(thio)cyanato-1,2,5-oxadiazoles of the formula (IV)

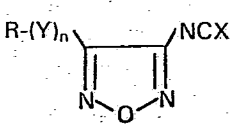

(IV)

in which

X, Y, R and n have the meanings given above in the case of formula Ia,

are reacted with amines of the formula (V)

$NHR^1R^2$ (V)

in which

$R^1$ and $R^2$ have the meanings given above in the case of formula Ia,

if appropriate in the presence of catalysts and if appropriate in the presence of diluents.

4. Agents for combating pests, characterised in that they contain at least one substituted furazan of the formula (Ia) according to claim 1.

5. Use of compounds of the formula (I)

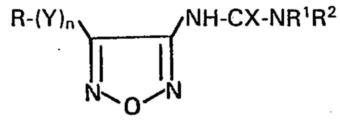

(I)

in which

R represents radicals which are optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkylcarbonyl, halogeno-$C_1$-$C_4$-alkoxycarbonyl, $C_1$-$C_4$-alkoxycarbonyl, $C_1$-$C_4$-alkoxycarbonyl-$C_1$-$C_2$-alkyl and/or $C_1$-$C_4$-alkoxycarbonyl-$C_1$-$C_4$-alkylthio and are chosen from the series comprising phenyl, pyridinyl, pyrimidinyl and 1,2,3-, 1,2,4- and 1,3,5-triazinyl,

$R^1$ represents radicals from the series comprising phenyl, pyridyl, pyrimidinyl and 1,2,3-, 1,2,4- and 1,3,5-triazinyl, which can be substituted by radicals from the series comprising halogen, nitro, cyano, $C_1$-$C_4$-alkyl, $C_1$-$C_4$-alkoxy, $C_1$-$C_6$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkyl-carbonyl, $C_1$-$C_4$-alkoxycarbonyl, halogeno-$C_1$-$C_4$-alkoxycarbonyl, $C_1$-$C_4$-alkoxycarbonyl-$C_1$-$C_2$-alkyl, $C_1$-$C_4$-alkoxycarbonyl-$C_1$-$C_4$-alkylthio, $C_1$-$C_4$-alkoxy-$C_1$-$C_4$-alkylthio, and/or a $C_1$-$C_3$-alkylene radical which contains 1 or 2 oxygen atoms and is optionally substituted by fluorine, chlorine and/or methyl, and/or a phenoxy or phenylthio radical, which can in turn be substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkyl-thio, $C_1$-$C_4$-alkoxy-$C_1$-$C_2$-alkyl, $C_1$-$C_4$-alkylthio-$C_1$-$C_2$-alkyl, $C_1$-$C_4$-alkylsulphonyl, $C_1$-$C_4$-alkoxycarbonyl and/or a $C_1$-$C_3$-alkylene radical which contains 1 or 2 oxygen atoms and is optionally substituted by fluorine, chlorine and/or methyl, and/or a pyridin-2-yloxy radical, which can in turn be substituted by halogen, $C_1$-$C_4$-alkyl, halogeno-$C_1$-$C_2$-alkoxy and/or halogeno-$C_1$-$C_2$-alkyl, and/or a pyrimidin-5-yloxy or 1,3,5-triazin-4-yloxy radical, which can in turn be substituted by $C_1$-$C_2$-alkyl, $C_1$-$C_2$-alkoxy, $C_1$-$C_2$-alkylthio, halogeno-$C_1$-$C_2$-alkyl, halogeno-$C_1$-$C_2$-alkoxy, halogeno-$C_1$-$C_2$-alkylthio, fluorine, chlorine and/or bromine,

$R^2$ represents hydrogen, an OH or COOH grouping, or radicals which are optionally substituted by fluorine, chlorine, $C_1$-$C_2$-alkyl, $C_1$-$C_2$-alkoxy, $C_1$-$C_2$-alkylthio, cyano and/or nitro and are chosen from the series comprising $C_1$-$C_4$-alkyl, $C_1$-$C_4$-alkoxy, $C_3$-$C_6$-cycloalkyl, $C_3$-$C_6$-cycloalkenyl with 1 or 2 double bonds, $C_1$-$C_4$-alkoxycarbonyl, $C_1$-$C_4$-alkoxythiocarbonyl, $C_1$-$C_4$-alkylsulphonyl, phenylsulphonyl, benzyl and phenylethyl,

Y represents oxygen or an $S(O)_m$, $-CR^3R^4$, CO or $NR^5$ grouping,

in which

m represents the numbers 0, 1 or 2 and

$R^3$, $R^4$ and $R^5$ represent hydrogen or $C_1$-$C_4$-alkyl,
n represents the numbers 0 or 1 and
X represents oxygen or sulphur,
for combating pests, in particular insects and acarids.

6. Method of combating pests, characterised in that compounds of the formula (I) according to claim 5 are allowed to act on the pests, preferably insects and acarids, or their environment.

7. Process for the preparation of agents for combating pests, characterised in that compounds of the formula (Ia) according to claim 1 are mixed with extenders and/or surface-active agents.

8. Compounds of the formula (IV)

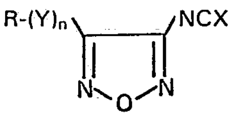

(IV)

in which
R represents radicals which are optionally substituted by halogen, nitro, cyano, $C_1$-$C_4$-alkyl, $C_1$-$C_4$-alkoxy, $C_1$-$C_4$-alkylthio, halogeno-$C_1$-$C_4$-alkyl, halogeno-$C_1$-$C_4$-alkoxy, halogeno-$C_1$-$C_4$-alkylthio, halogeno-$C_1$-$C_4$-alkylcarbonyl, $C_1$-$C_4$-alkylcarbonyl, halogeno-$C_1$-$C_4$-alkoxycarbonyl, $C_1$-$C_4$-alkoxycarbonyl, $C_1$-$C_4$-alkoxycarbonyl-$C_1$-$C_2$-alkyl and/or $C_1$-$C_4$-alkoxycarbonyl-$C_1$-$C_4$-alkylthio and are chosen from the series comprising phenyl, pyridinyl, pyrimidinyl and 1,2,3-, 1,2,4- and 1,3,5-triazinyl,
Y represents oxygen or an $S(O)_m$, $-CR^3R^4$, CO or $NR^5$ grouping,
in which
m represents the numbers 0, 1 or 2 and
$R^3$, $R^4$ and $R^5$ represent hydrogen or $C_1$-$C_4$-alkyl,
n represents the numbers 0 or 1 and
X represents oxygen or sulphur.

## Revendications

1. Furazanes substitués de formule (Ia):

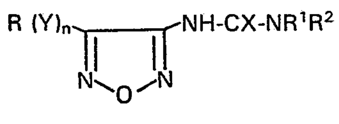

(Ia)

dans laquelle
R représente des radicaux choisis parmi la série comprenant le groupe phényle, le groupe pyridinyle, le groupe pyrimidinyle, les groupes 1,2,3-, 1,2,4- et 1,3,5-triazinyle éventuellement substitués par un atome d'halogène, un groupe nitro, un groupe cyano, un groupe alkyle en $C_1$-$C_4$, un groupe alkoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe halogéno-alcoxy(en $C_1$-$C_4$)carbonyle, un groupe alcoxy(en $C_1$-$C_4$)carbonyle, un groupe alcoxy(en $C_1$-$C_4$)carbonyl-alkyle en $C_1$-$C_2$ et/ou un groupe alcoxy(en $C_1$-$C_4$)carbonyl-alkyl(en $C_1$-$C_4$)thio,

$R^1$ représente des radicaux choisis parmi la série comprenant le groupe phényle, le groupe pyridyle, le groupe pyrimidinyle, les groupes 1,2,3-, 1,2,4- et 1,3,5-triazinyle qui peuvent être substitués par des radicaux choisis parmi la série comprenant un atome d'halogène, un groupe nitro, un groupe cyano, un groupe alkyle en $C_1$-$C_4$, un groupe alcoxy en $C_1$-$C_4$, un groupe alkyl(en $C_1$-$C_6$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe alcoxy(en $C_1$-$C_4$)carbonyle, un groupe halogéno-alkoxy(en $C_1$-$C_4$)carbonyle, un groupe alcoxy(en $C_1$-$C_4$)carbonyl-alkyle en $C_1$-$C_2$, un groupe alcoxy(en $C_1$-$C_4$)carbonyl-alkyl(en $C_1$-$C_4$)thio, un groupe alcoxy(en $C_1$-$C_4$)-alkyl(en $C_1$-$C_4$)thio et/ou un groupe alkylène en $C_1$-$C_3$ contenant 1 ou 2 atomes d'oxygène et éventuellement substitué par un atome de fluor, un atome de chlore et/ou un groupe méthyle, et/ou un groupe phénoxy ou un groupe phénylthio qui, à son tour, peut être substitué par un atome d'halogène, un groupe nitro, un groupe cyano, un groupe alkyle en $C_1$-$C_4$, un groupe alcoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe alcoxy(en $C_1$-$C_4$)alkyle en $C_1$-$C_2$, un groupe alkyl(en $C_1$-$C_4$)thio-alkyle en $C_1$-$C_2$, un groupe alkyl(en $C_1$-$C_4$)sulfonyle, un groupe alcoxy(en $C_1$-$C_4$)carbonyle et/ou un groupe alkylène en $C_1$-$C_3$ contenant 1 ou 2 atomes d'oxygène et pouvant éventuellement être substitué par un atome de fluor, un atome de chlore et/ou un groupe méthyle, et/ou un groupe pyridin-2-yloxy qui, à son tour, peut être substitué par un atome d'halogène, un groupe alkyle en $C_1$-$C_4$, un groupe halogéno-alcoxy en $C_1$-$C_2$ et/ou un groupe halogéno-alkyle en $C_1$-$C_2$, et/ou un groupe pyrimidin-5-yloxy ou 1,3,5-triazin-4-yloxy qui, à son tour, peut être substitué par un groupe alkyle en $C_1$-$C_2$, un groupe alcoxy en $C_1$-$C_2$, un groupe alkyl(en $C_1$-$C_2$)thio, un groupe halogéno-alkyle en $C_1$-$C_2$, un groupe halogéno-alcoxy en $C_1$-$C_2$, un groupe halogéno-alkyl(en $C_1$-$C_2$)thio, un atome de fluor, un atome de chlore et/ou un atome de brome,

$R^2$ représente un atome d'hydrogène, un groupe OH ou COOH ou des radicaux choisis parmi la série comprenant un groupe alkyle en $C_1$-$C_4$, un groupe alcoxy en $C_1$-$C_4$, un groupe cycloalkyle en $C_2$-$C_6$, un groupe cycloalcényle en $C_3$-$C_6$ comportant une ou deux doubles liaisons, un groupe alcoxy(en $C_1$-$C_4$)carbonyle, un groupe alcoxy(en $C_1$-$C_4$)thiocarbonyle, un groupe alkyl(en $C_1$-$C_4$)sulfonyle, un groupe phénylsulfonyle, un groupe benzyle et un groupe phényléthyle, éventuellement substitués par un atome de fluor, un atome de chlore, un groupe alkyle en $C_1$-$C_2$, un groupe alcoxy en $C_1$-$C_2$, un groupe alkyl(en $C_1$-$C_2$)thio, un groupe cyano et/ou un groupe nitro,

Y représente l'oxygène, un groupe $S(O)_m$-, $-CR^3R^4$-, CO- ou $NR^5$- où m représente les nombres 0, 1 ou 2,

$R^3$, $R^4$ et $R^5$ représentent chacun un atome d'hydrogène ou un groupe alkyle en $C_1$-$C_4$,

n représente les nombres 0 ou 1, et

X représente l'oxygène ou le soufre,

et, lorsque

R représente le groupe phényle,

$R^1$ représente le groupe phényle,

$R^2$ représente l'hydrogène, et

X représente l'oxygène ou le soufre,

dans ces cas,

n représente le nombre 1, ou

lorsque

n représente le nombre 0,

R représente le groupe 4-bromophényle,

$R^1$ représente le groupe 4-chlorophényle, et

$R^2$ représente l'hydrogène,

dans ce cas,

X représente le soufre.

2. Furazanes substitués de formule (Ia) selon la revendication 1, dans laquelle

R représente des radicaux choisis parmi la série comprenant le groupe phényle, le groupe pyridin-2-yle, le groupe pyrimidin-2-yle, le groupe 1,2,4-triazin-2-yle et le groupe 1,3,5-triazin-2-yle qui peuvent être substitués par un atome de fluor, un atome de chlore, un atome de brome, un groupe cyano, un groupe nitro, un groupe méthyle, un groupe éthyle, un groupe méthoxy, un groupe éthoxy, un groupe méthylthio, un groupe trifluorométhyle, un groupe trifluorométhoxy, un groupe trifluorométhylthio, un groupe méthylcarbonyle, un groupe méthoxycarbonyle, un groupe éthoxycarbonyle, un groupe n-butoxycarbonyle, un groupe tert.-butoxycarbonyle, un groupe méthoxycarbonylméthyle, un groupe éthoxycarbonylméthyle, un groupe méthoxycarbonylméthylthio et/ou un groupe éthoxycarbonylméthylthio,

$R^1$ représente un groupe phényle qui peut être substitué par des radicaux choisis parmi la série comprenant un atome de fluor, un atome de chlore, un atome de brome, un groupe nitro, un groupe cyano, un groupe méthoxy, un groupe éthoxy, un groupe méthyle, un groupe éthyle, un groupe n-propyle, un groupe i-propyle, un groupe n-butyle, un groupe i-butyle, un groupe sec.-butyle, un groupe tert.-butyle, un groupe méthylthio, un groupe éthylthio, un groupe trifluorométhyle, un groupe trifluorométhoxy, un groupe difluorométhyle, un groupe difluoroéthyle, un groupe pentafluoroéthyle, un groupe difluorométhoxy, un groupe chlorodifluorométhoxy, un groupe chlorotrifluoréthoxy, un groupe tétrafluoréthoxy, un groupe dichlorodifluoréthoxy, un groupe difluorotrichloréthoxy, un groupe hexafluoropropoxy, un groupe trifluorométhylthio, un groupe difluorométhylthio, un groupe chlorodifluorométhylthio, un groupe chlorotrifluoréthylthio, un groupe hexafluoropropylthio, un groupe méthoxycarbonyle, un groupe éthoxycarbonyle, un groupe n-propoxycarbonyle, un groupe i-propoxycarbonyle, un groupe n-butoxycarbonyle, un groupe i-butoxycarbonyle, un groupe sec.-butoxycarbonyle, un groupe tert.-butoxycarbonyle, un groupe trifluoréthoxycarbonyle, un groupe méthylcarbonyle, un groupe méthoxycarbonylméthyle, un groupe méthoxycarbonyléthyle, un groupe éthoxycarbonylméthyle, un groupe difluorométhylènedioxy, un groupe éthylène-dioxy, un groupe chlorotrifluoréthylènedioxy, un groupe difluoréthylène-dioxy, un groupe méthylène-dioxy, un groupe trifluoréthylène-dioxy, un groupe difluorométhylène-oxydifluorométhylène-oxy, un groupe tétrafluoréthylène-dioxy, un groupe 2-méthyl-1,1,2-trifluoréthylène-dioxy, un groupe 2,2-diméthyléthylène-oxy et/ou un groupe phénoxy qui, à son tour, est éventuellement substitué par un atome de chlore, un atome de brome, un groupe nitro, un groupe cyano, un groupe méthyle, un groupe éthyle, un groupe n-propyle, un groupe i-propyle, un groupe n-butyle, un groupe i-butyle, un groupe sec.-butyle, un groupe tert.-butyle, un groupe méthoxy, un groupe éthoxy, un groupe n-propoxy, un groupe i-propoxy, un groupe n-butoxy, un groupe i-butoxy, un groupe sec.-butoxy, un groupe tert.-butoxy, un groupe trifluorométhyle, un groupe trifluorométhoxy, un groupe difluorométhyle, un groupe difluoréthyle, un groupe pentafluoréthyle, un groupe difluorométhoxy, un groupe chlorodifluorométhoxy, un groupe chlorotrifluoréthoxy, un groupe tétrafluoréthoxy, un groupe dichlorodifluoréthoxy, un groupe difluorotrichloréthoxy, un groupe hexafluoropropoxy, un groupe trifluorométhylthio, un groupe difluorométhylthio, un groupe chlorodifluorométhylthio, un groupe chlorotrifluoréthylthio, un groupe hexafluoropropylthio, un groupe méthoxyméthyle, un groupe éthoxyméthyle, un groupe méthylthiométhyle, un groupe éthylthiométhyle, un groupe méthoxycarbonyle, un groupe éthoxycarbonyle, un groupe n-propoxycarbonyle, un groupe i-propoxycarbonyle, un groupe n-butoxycarbonyle, un groupe i-butoxycarbonyle, un groupe sec.-butoxycarbonyle, un groupe tert.-butoxycarbonyle, un groupe difluorométhylène-dioxy, un groupe éthylène-dioxy, un groupe chlorotrifluoréthylène-dioxy, un groupe difluoréthylène-dioxy, un groupe méthylène-dioxy, un groupe trifluoréthylène-dioxy, un groupe difluorométhylène-oxydifluorométhylène-oxy, un groupe tétrafluoréthylène-dioxy, un groupe 2-méthyl-1,1,2-trifluoréthylène-dioxy et/ou un groupe 2,2-diméthyléthylène-oxy, et/ou un groupe pyridin-2-yloxy qui, à son tour, est éventuellement substitué par un atome de chlore, un atome de brome, un groupe méthyle, un groupe trifluorométhyle et/ou un groupe trifluorométhoxy, et/ou un groupe pyrimidin-5-yloxy qui, à son tour, est éventuellement substitué par un groupe méthyle, un groupe méthoxy, un groupe méthylthio, un groupe trifluorométhyle, un groupe trifluorométhoxy, un groupe trifluorométhylthio, un atome de chlore et/ou un atome de brome,

$R^2$ représente un atome d'hydrogène, un groupe méthyle, un groupe éthyle, un groupe n-propyle, un groupe i-propyle, un groupe n-butyle, un groupe i-butyle, ou un groupe sec.-butyle,

Y représente un atome d'oxygène, un groupe $S(O)_m$-, un groupe $CR^3R^4$-, un groupe CO- ou un groupe $NR^5$ où m représente 0, 1 ou 2,

$R^3$, $R^4$ et $R^5$ représentent chacun un atome d'hydrogène ou un groupe méthyle

n représente les nombres 0 ou 1, et

X représente l'oxygène ou le soufre,

et, lorsque

R représente le groupe phényle,

$R^1$ représente le groupe phényle,

$R^2$ représente l'hydrogène, et

X représente l'oxygène ou le soufre,
dans ce cas,
n représente le nombre 1,
ou lorsque
n représente le nombre 0,
R représente le groupe 4-bromophényle,
$R^1$ représente le groupe 4-chlorophényle, et
$R^2$ représente l'hydrogène,
dans ce cas
X représente le soufre.

3. Procédé de préparation des nouveaux furazanes substitués de formule (Ia):

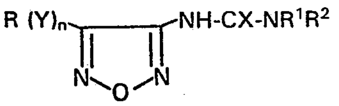

(Ia)

dans laquelle

R représente des radicaux choisis parmi la série comprenant le groupe phényle, le groupe pyridinyle, le groupe pyrimidinyle, les groupes 1,2,3-, 1,2,4- et 1,3,5-triazinyle éventuellement substitués par un atome d'halogène, un groupe nitro, un groupe cyano, un groupe alkyle en $C_1$-$C_4$, un groupe alkoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe halogéno-alcoxy(en $C_1$-$C_4$)carbonyle, un groupe alcoxy(en $C_1$-$C_4$)carbonyle, un groupe alcoxy(en $C_1$-$C_4$)carbonyl-alkyle en $C_1$-$C_2$ et/ou un groupe alcoxy(en $C_1$-$C_4$)carbonyl-alkyl(en $C_1$-$C_4$)thio,

$R^1$ représente des radicaux choisis parmi la série comprenant le groupe phényle, le groupe pyridyle, le groupe pyrimidinyle, les groupes 1,2,3-, 1,2,4- et 1,3,5-triazinyle qui peuvent être substitués par des radicaux choisis parmi la série comprenant un atome d'halogène, un groupe nitro, un groupe cyano, un groupe alkyle en $C_1$-$C_4$, un groupe alcoxy en $C_1$-$C_4$, un groupe alkyl(en $C_1$-$C_6$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe alcoxy(en $C_1$-$C_4$)carbonyle, un, groupe halogéno-alkoxy(en $C_1$-$C_4$)carbonyle, un groupe alcoxy(en $C_1$-$C_4$)carbonyl-alkyle en $C_1$-$C_2$, un groupe alcoxy(en $C_1$-$C_4$)carbonyl-alkyl(en $C_1$-$C_4$)thio, un groupe alcoxy(en $C_1$-$C_4$)-alkyl(en $C_1$-$C_4$)thio et/ou un groupe alkylène en $C_1$-$C_3$ contenant 1 ou 2 atomes d'oxygène et éventuellement substitué par un atome de fluor, un atome de chlore et/ou un groupe méthyle, et/ou un groupe phénoxy ou un groupe phénylthio qui, à son tour, peut être substitué par un atome d'halogène, un groupe nitro, un groupe cyano, un groupe alkyle en $C_1$-$C_4$, un groupe alcoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe alcoxy(en $C_1$-$C_4$)alkyle en $C_1$-$C_2$, un groupe alkyl(en $C_1$-$C_4$)thio-alkyle en $C_1$-$C_2$, un groupe alkyl(en $C_1$-$C_4$)sulfonyle, un groupe alcoxy(en $C_1$-$C_4$)carbonyle et/ou un groupe alkylène en $C_1$-$C_3$ contenant 1 ou 2 atomes d'oxygène et pouvant éventuellement être substitué par un atome de fluor, un atome de chlore et/ou un groupe méthyle, et/ou un groupe pyridin-2-yloxy qui, à son tour, peut être substitué par un atome d'halogène, un groupe alkyle en $C_1$-$C_4$, un groupe halogéno-alcoxy en $C_1$-$C_2$ et/ou un groupe halogéno-alkyle en $C_1$-$C_2$, et/ou un groupe pyrimidin-5-yloxy ou 1,3,5-triazin-4-yloxy qui, à son tour, peut être substitué par un groupe alkyle en $C_1$-$C_2$, un groupe alcoxy en $C_1$-$C_2$, un groupe alkyl(en $C_1$-$C_2$)thio, un groupe halogéno-alkyle en $C_1$-$C_2$, un groupe halogéno-alcoxy en $C_1$-$C_2$, un groupe halogéno-alkyl(en $C_1$-$C_2$)thio, un atome de fluor, un atome de chlore et/ou un atome de brome,

$R^2$ représente un atome d'hydrogène, un groupe OH ou COOH ou encore des radicaux choisis parmi la série comprenant un groupe alkyle en $C_1$-$C_4$, un groupe alcoxy en $C_1$-$C_4$, un groupe cycloalkyle en $C_2$-$C_6$, un groupe cycloalcényle en $C_3$-$C_6$ comportant une ou deux doubles liaisons, un groupe alcoxy(en $C_1$-$C_4$)carbonyle, un groupe alcoxy(en $C_1$-$C_4$)thiocarbonyle, un groupe alkyl(en $C_1$-$C_4$)sulfonyle, un groupe phénylsulfonyle, un groupe benzyle et un groupe phényléthyle, éventuellement substitués par un atome de fluor, un atome de chlore, un groupe alkyle en $C_1$-$C_2$, un groupe alcoxy en $C_1$-$C_2$, un groupe alkyl(en $C_1$-$C_2$)thio, un groupe cyano et/ou un groupe nitro,

Y représente l'oxygène, un groupe $S(O)_m$-, un groupe -$CR^3R^4$-, un groupe CO- ou un groupe $NR^5$- où m représente les nombres 0, 1 ou 2,

$R^3$, $R^4$ et $R^5$ représentent chacun un atome d'hydrogène ou un groupe alkyle en $C_1$-$C_4$,

n représente les nombres 0 ou 1, et
X représente l'oxygène ou le soufre,
et, lorsque
R représente le groupe phényle,
$R^1$ représente le groupe phényle,
$R^2$ représente l'hydrogène, et
X représente l'oxygène ou le soufre,
dans ces cas,
n représente le nombre 1, ou
lorsque
n représente le nombre 0,
R représente le groupe 4-bromophényle,
$R^1$ représente le groupe 4-chlorophényle, et
$R^2$ représente l'hydrogène,
dans ce cas,
X représente le soufre,
caractérisé en ce que

a) on fait réagir des 3-amino-1,2,5-oxadiazoles de formule (II):

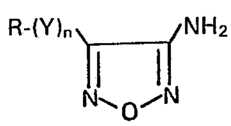

(II)

dans laquelle

Y, R et n ont les significations indiquées pour la formule Ia,

avec des iso(thio)cyanates de formule (III):

$R^1NCX$ (III)

dans laquelle

$R^1$ et X ont les significations indiquées pour la formule Ia,

éventuellement en présence de catalyseurs et éventuellement en présence de diluants, ou

b) on fait réagir 3-iso(thio)cyanato-1,2,5-oxadiazoles de formule (IV):

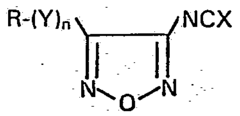

(IV)

dans laquelle

X, Y, R et n ont les significations indiquées ci-dessus pour la formule Ia,

avec des amines de formule (V):

$NHR^1R^2$ (V)

dans laquelle

$R^1$ et $R^2$ ont les significations indiquées ci-dessus pour la formule Ia,

éventuellement en présence de catalyseurs et éventuellement en présence de diluants.

4. Agents parasiticides, caractérisés en ce qu'ils contiennent au moins un furazane substitué de formule (Ia) selon la revendication 1.

5. Utilisation de composés de formule (I):

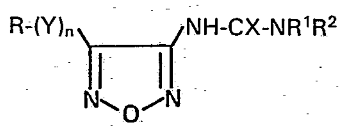

(I)

dans laquelle

R représente des radicaux choisis parmi la série comprenant le groupe phényle, le groupe pyridinyle, le groupe pyrimidinyle, les groupes 1,2,3-, 1,2,4- et 1,3,5-triazinyle éventuellement substitués par un atome d'halogène, un groupe nitro, un groupe cyano, un groupe alkyle en $C_1$-$C_4$, un groupe alkoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe halogéno-alcoxy(en $C_1$-$C_4$)carbonyle, un groupe alcoxy(en $C_1$-$C_4$)carbonyle, un groupe alcoxy(en $C_1$-$C_4$)carbonyl-alkyle en $C_1$-$C_2$ et/ou un groupe alcoxy(en $C_1$-$C_4$)carbonyl-alkyl(en $C_1$-$C_4$)thio,

$R^1$ représente des radicaux choisis parmi la série comprenant le groupe phényle, le groupe pyridyle, le groupe pyrimidinyle, les groupes 1,2,3-, 1,2,4- et 1,3,5-triazinyle qui peuvent être substitués par des radicaux choisis parmi la série comprenant un atome d'halogène, un groupe nitro, un groupe cyano, un groupe alkyle en $C_1$-$C_4$, un groupe alcoxy en $C_1$-$C_4$, un groupe alkyl(en $C_1$-$C_6$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe alcoxy(en $C_1$-$C_4$)carbonyle, un groupe halogéno-alkoxy(en $C_1$-$C_4$)carbonyle, un groupe alcoxy(en $C_1$-$C_4$)carbonyl-alkyle en $C_1$-$C_2$, un groupe alcoxy(en $C_1$-$C_4$)carbonyl-alkyl(en $C_1$-$C_4$)thio, un groupe alcoxy(en $C_1$-$C_4$)-alkyl(en $C_1$-$C_4$)thio et/ou un groupe alkylène en $C_1$-$C_3$ contenant 1 ou 2 atomes d'oxygène et éventuellement substitué par un atome de fluor, un atome de chlore et/ou un groupe méthyle, et/ou un groupe phénoxy ou un groupe phénylthio qui, à son tour, peut être substitué par un atome d'halogène, un groupe nitro, un groupe cyano, un groupe alkyle en $C_1$-$C_4$, un groupe alcoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe alcoxy(en $C_1$-$C_4$)alkyle en $C_1$-$C_2$, un groupe alkyl(en $C_1$-$C_4$)thio-alkyle en $C_1$-$C_2$, un groupe alkyl(en $C_1$-$C_4$)sulfonyle, un groupe alcoxy(en $C_1$-$C_4$)carbonyle et/ou un groupe alkylène en $C_1$-$C_3$ contenant 1 ou 2 atomes d'oxygène et pouvant éventuellement être substitué par un atome de fluor, un atome de chlore et/ou un groupe méthyle, et/ou un groupe pyridin-2-yloxy qui, à son tour, peut être substitué par un atome d'halogène, un groupe alkyle en $C_1$-$C_4$, un groupe halogéno-alcoxy en $C_1$-$C_2$ et/ou un groupe halogéno-alkyle en $C_1$-$C_2$, et/ou un groupe pyrimidin-5-yloxy ou 1,3,5-triazin-4-yloxy qui, à son tour, peut être substitué par un groupe alkyle en $C_1$-$C_2$, un groupe alcoxy en $C_1$-$C_2$, un groupe alkyl(en $C_1$-$C_2$)thio, un groupe halogéno-alkyle en $C_1$-$C_2$, un groupe halogéno-alcoxy en $C_1$-$C_2$, un groupe halogéno-alkyl(en $C_1$-$C_2$)thio, un atome de fluor, un atome de chlore et/ou un atome de brome,

$R^2$ représente un atome d'hydrogène, un groupe OH ou COOH ou encore des radicaux choisis parmi la série comprenant un groupe alkyle en $C_1$-$C_4$, un groupe alcoxy en $C_1$-$C_4$, un groupe cycloalkyle en $C_2$-$C_6$, un groupe cycloalcényle en $C_3$-$C_6$ comportant une ou deux doubles liaisons, un groupe alcoxy(en $C_1$-$C_4$)carbonyle, un groupe alcoxy(en $C_1$-$C_4$)thiocarbonyle, un groupe alkyl(en $C_1$-$C_4$)sulfonyle, un groupe phénylsulfonyle, un groupe benzyle et un groupe phényléthyle, éventuellement substitués par un atome de fluor, un atome de chlore, un groupe alkyle en $C_1$-$C_2$, un groupe alcoxy en $C_1$-$C_2$, un groupe alkyl(en $C_1$-$C_2$)thio, un groupe cyano et/ou un groupe nitro,

Y représente l'oxygène, un groupe $S(O)_m$-, un groupe $-CR^3R^4$-, un groupe CO- ou un groupe $NR^5$- où m représente les nombres 0, 1 ou 2,

$R^3$, $R^4$ et $R^5$ représentent chacun un atome d'hydrogène ou un groupe alkyle en $C_1$-$C_4$,

n représente les nombres 0 ou 1, et

X représente l'oxygène ou le soufre,

pour combattre les parasites, en particulier, les insectes et les acariens.

6. Procédé en vue de combattre les parasites, caractérisé en ce qu'on fait agir des composés de formule (I) selon la revendication 5 sur les parasites, de préférence, les insectes et les acariens, ou leurs biotopes.

7 Procédé de préparation d'agents parasiticides, caractérisé en ce qu'on mélange des composés de formule (Ia) selon la revendication 1 avec des diluants et/ou des agents tensio-actifs.

8. Composés de formule (IV):

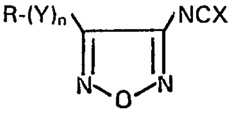

(IV)

dans laquelle

R représente des radicaux choisis parmi la série comprenant le groupe phényle, le groupe pyridinyle, le groupe pyrimidinyle, les groupes 1,2,3-, 1,2,4- et 1,3,5-triazinyle éventuellement substitués par un atome d'halogène, un groupe nitro, un groupe cyano, un groupe alkyle en $C_1$-$C_4$, un groupe alkoxy en $C_1$-$C_4$, un groupe alkyl(en $C_1$-$C_4$)thio, un groupe halogéno-alkyle en $C_1$-$C_4$, un groupe halogéno-alcoxy en $C_1$-$C_4$, un groupe halogéno-alkyl(en $C_1$-$C_4$)thio, un groupe halogéno-alkyl(en $C_1$-$C_4$)carbonyle, un groupe alkyl(en $C_1$-$C_4$)carbonyle, un groupe halogéno-alcoxy(en $C_1$-$C_4$)carbonyle, un groupe alcoxy(en $C_1$-$C_4$)carbonyle, un groupe alcoxy(en $C_1$-$C_4$)carbonyl-alkyle en $C_1$-$C_2$ et/ou un groupe alcoxy(en $C_1$-$C_4$)carbonyl-alkyl(en $C_1$-$C_4$)thio,

Y représente l'oxygène, un groupe $S(O)_m$-, un groupe $-CR^3R^4-$, un groupe CO- ou un groupe $NR^5$- où m représente les nombres 0, 1 ou 2,

$R^3$, $R^4$ et $R^5$ représentent chacun un atome d'hydrogène ou un groupe alkyle en $C_1$-$C_4$,

n représente les nombres 0 ou 1, et

X représente l'oxygène ou le soufre.